# EUROPEAN PATENT SPECIFICATION

(11) **EP 3 129 037 B1**
(45) Date of publication and mention of the grant of the patent: **13.11.2019**
(21) Application number: 15720276.3
(22) Date of filing: 09.04.2015
(51) Int. Cl.: A61K 35/768, A61K 31/282, A61K 31/4745, A61P 35/00

(54) **POXVIRAL ONCOLYTIC VECTORS**
ONKOLYTISCHE POXVIRUS-VEKTOREN
VECTEURS ONCOLYTIQUES POXVIRAUX

(30) Priority: 10.04.2014 US 201414249713
(43) Date of publication of application: 15.02.2017
(73) Proprietor: TRANSGENE, 67400 Illkirch Graffenstaden (FR)
(72) Inventor: ERBS, Philippe, F-67120 Molsheim (FR); FOLOPPE, Johann, F-67115 Plobsheim (FR)
(74) Representative: Vossius & Partner Patentanwälte Rechtsanwälte mbB
(86) International application number: PCT/EP2015/057654
(87) International publication number: WO 2015/155263

(56) References cited:
- WO-A1-2009/011924
- WO-A1-2009/065546
- WO-A1-2011/003194
- GUSE KILIAN ET AL: "Oncolytic vaccinia virus for the treatment of cancer", EXPERT OPINION ON BIOLOGICAL THERAPY, vol. 11, no. 5, 1 May 2011 (2011-05-01), pages 1-14, XP002689199, INFORMA HEALTHCARE, UK ISSN: 1471-2598
- DON B GAMMON ET AL: "Vaccinia Virus-Encoded Ribonucleotide Reductase Subunits Are Differentially Required for Replication and Pathogenesis", PLOS PATHOGENS, vol. 6, no. 7, E1000984, 8 July 2010 (2010-07-08), XP008150295, PUBLIC LIBRARY OF SCIENCE, SAN FRANCISCO, CA, US ISSN: 1553-7366, DOI: 10.1371/JOURNAL.PPAT.1000984
- Anonymous: "Transgene Announces that Clinical Data with Pexa-Vec will be Presented at the 2013 American Society of Clinical Oncology Annual Meeting", Transgene , 15 May 2013 (2013-05-15), pages 1-3, XP055201878, Retrieved from the Internet: URL:http://www.transgene.fr/wp-content/upl oads/PR/229_en.pdf [retrieved on 2015-07-13]
- M F ZIAUDDIN ET AL: "TRAIL gene-armed oncolytic poxvirus and oxaliplatin can work synergistically against colorectal cancer", GENE THERAPY, vol. 17, no. 4, 25 February 2010 (2010-02-25), pages 550-559, XP055201128, ISSN: 0969-7128, DOI: 10.1038/gt.2010.5
- YU YONG A ET AL: "Regression of human pancreatic tumor xenografts in mice after a single systemic injection of recombinant vaccinia virus GLV-1h68", MOLECULAR CANCER THERAPEUTICS, vol. 8, no. 1, 1 January 2009 (2009-01-01) , pages 141-151, XP008101257, AMERICAN ASSOCIATION OF CANCER RESEARCH, US ISSN: 1535-7163, DOI: 10.1158/1535-7163.MCT-08-0533
- Declaration by Philippe Erbs
- "8 th International Conference on Oncolytic Virus Therapeutics 2014 - April 10-13, 2014 Lincoln College & Examination Schools - Oxford, United Kingdom", HUMAN GENE THERAPY, vol. 25, no. 12, 1 December 2014 (2014-12-01), pages A1-A32, XP055253040, US ISSN: 1043-0342, DOI: 10.1089/hum.2014.2538.abstracts
- J Foloppe ET AL: "Oncolytic vaccinia virus TG6002 demonstrates potent efficiency in pancreatic tumor alone and in combination with standard chemotherapies", XXI International Poxvirus, Asfavirus and Iridovirus Conference, 1 July 2016 (2016-07-01), XP55434402, England Retrieved from the Internet: URL:https://www.transgene.fr/wp-content/up loads/2016/07/160701-Poster_Poxvirus_congr ess_TG6002.pdf [retrieved on 2017-12-12]
- SANCHEZ-SAMPEDRO LUCAS ET AL: "Attenuated and Replication-Competent Vaccinia Virus Strains M65 and M101 with Distinct Biology and Immunogenicity as Potential Vaccine Candidates against Pathogens", JOURNAL OF VIROLOGY, vol. 87, no. 12, June 2013 (2013-06), pages 6955-6974,
- POTTS KYLE G ET AL: "Deletion of F4L (ribonucleotide reductase) in vaccinia virus produces a selective oncolytic virus and promotes anti-tumor immunity with superior safety in bladder cancer models", EMBO MOLECULAR MEDICINE, vol. 9, no. 5, May 2017 (2017-05), pages 638-654,
- Anonymous: "Squirrelpox virus J2R / thymidine kinase", , 7 January 2014 (2014-01-07), Retrieved from the Internet: URL:https://www.viprbrc.org/brc/viprDetail s.spg?ncbiProteinId=YP_008658464&decorator =pox&context=1524219001996/ [retrieved on 2018-07-30]
- Anonymous: "Cotia virus J2R /Thymidine kinase", , 20 July 2009 (2009-07-20), Retrieved from the Internet: URL:https://www.viprbrc.org/brc/viprDetail s.spg?ncbiProteinId=ADL60426&decorator=pox &context=1524219001996/ [retrieved on 2018-07-30]
- Anonymous: "Camelpox virus I4L / ribonucleoside-diphosphate reductase large subunit", , 12 February 2015 (2015-02-12), Retrieved from the Internet: URL:https://www.viprbrc.org/brc/viprDetail s.spg?ncbiProteinId=AKU40439&decorator=pox &context=1524218135559/ [retrieved on 2018-07-30]
- Anonymous: "Monkeypox virus I4L / ribonucleoside-diphosphate reductase large subunit", , 23 February 2015 (2015-02-23), Retrieved from the Internet: URL:https://www.viprbrc.org/brc/viprDetail s.spg?ncbiProteinId=AKG51040&decorator=pox &context=1524218135559/ [retrieved on 2018-07-30]
- C. J. Mcinnes: "Genomic characterization of a novel poxvirus contributing to the decline of the red squirrel (Sciurus vulgaris) in the UK", JOURNAL OF GENERAL VIROLOGY., vol. 87, no. 8, 1 August 2006 (2006-08-01) , pages 2115-2125, XP055496123, GB ISSN: 0022-1317, DOI: 10.1099/vir.0.81966-0
- ANTOINE G ET AL: "The Complete Genomic Sequence of the Modified Vaccinia Ankara Strain: Comparison with Other Orthopoxviruses", VIROLOGY, ELSEVIER, AMSTERDAM, NL, vol. 244, no. 2, 10 May 1998 (1998-05-10), pages 365-396, XP004445824, ISSN: 0042-6822, DOI: 10.1006/VIRO.1998.9123

### SEQUENCE LISTING

<110> TRANSGENE S.A.
<120> Poxviral oncolytic vectors
<130> Y1411 PCT S3
<150> US14/249,713
   <151> 2014-04-10
<160> 18
<170> BiSSAP 1.2
<210> 1
   <211> 373
   <212> PRT
   <213> Artificial Sequence
<220>
   <223> derived from Saccharomyces cerevisiae
<400> 1
   Met Val Thr Gly Gly Met Ala Ser Lys Trp Asp Gln Lys Gly Met Asp
   1               5                   10                  15
   Ile Ala Tyr Glu Glu Ala Ala Leu Gly Tyr Lys Glu Gly Gly Val Pro
               20                  25                  30
   Ile Gly Gly Cys Leu Ile Asn Asn Lys Asp Gly Ser Val Leu Gly Arg
           35                  40                  45
   Gly His Asn Met Arg Phe Gln Lys Gly Ser Ala Thr Leu His Gly Glu
       50                  55                  60
   Ile Ser Thr Leu Glu Asn Cys Gly Arg Leu Glu Gly Lys Val Tyr Lys
   65                  70                  75                  80
   Asp Thr Thr Leu Tyr Thr Thr Leu Ser Pro Cys Asp Met Cys Thr Gly
                   85                  90                  95
   Ala Ile Ile Met Tyr Gly Ile Pro Arg Cys Val Val Gly Glu Asn Val
               100                 105                 110
   Asn Phe Lys Ser Lys Gly Glu Lys Tyr Leu Gln Thr Arg Gly His Glu
           115                 120                 125
   Val Val Val Val Asp Asp Glu Arg Cys Lys Lys Ile Met Lys Gln Phe
       130                 135                 140
   Ile Asp Glu Arg Pro Gln Asp Trp Phe Glu Asp Ile Gly Glu Ala Ser
   145                 150                 155                 160
   Glu Pro Phe Lys Asn Val Tyr Leu Leu Pro Gln Thr Asn Gln Leu Leu
                   165                 170                 175
   Gly Leu Tyr Thr Ile Ile Arg Asn Lys Asn Thr Thr Arg Pro Asp Phe
               180                 185                 190
   Ile Phe Tyr Ser Asp Arg Ile Ile Arg Leu Leu Val Glu Glu Gly Leu
           195                 200                 205
   Asn His Leu Pro Val Gln Lys Gln Ile Val Glu Thr Asp Thr Asn Glu
       210                 215                 220
   Asn Phe Glu Gly Val Ser Phe Met Gly Lys Ile Cys Gly Val Ser Ile
   225                 230                 235                 240
   Val Arg Ala Gly Glu Ser Met Glu Gln Gly Leu Arg Asp Cys Cys Arg
                   245                 250                 255
   Ser Val Arg Ile Gly Lys Ile Leu Ile Gln Arg Asp Glu Glu Thr Ala
               260                 265                 270
   Leu Pro Lys Leu Phe Tyr Glu Lys Leu Pro Glu Asp Ile Ser Glu Arg
           275                 280                 285
   Tyr Val Phe Leu Leu Asp Pro Met Leu Ala Thr Gly Gly Ser Ala Ile
       290                 295                 300
   Met Ala Thr Glu Val Leu Ile Lys Arg Gly Val Lys Pro Glu Arg Ile
   305                 310                 315                 320
   Tyr Phe Leu Asn Leu Ile Cys Ser Lys Glu Gly Ile Glu Lys Tyr His
                   325                 330                 335
   Ala Ala Phe Pro Glu Val Arg Ile Val Thr Gly Ala Leu Asp Arg Gly
               340                 345                 350
   Leu Asp Glu Asn Lys Tyr Leu Val Pro Gly Leu Gly Asp Phe Gly Asp
           355                 360                 365
   Arg Tyr Tyr Cys Val
       370
<210> 2
   <211> 373
   <212> PRT
   <213> Artificial Sequence
<220>
   <223> derived from Saccharomyces cerevisiae
<400> 2
   Met Val Thr Gly Gly Met Ala Ser Lys Trp Asp Gln Lys Gly Met Asp
   1               5                   10                  15
   Ile Ala Tyr Glu Glu Ala Ala Leu Gly Tyr Lys Glu Gly Gly Val Pro
               20                  25                  30
   Ile Gly Gly Cys Leu Ile Asn Asn Lys Asp Gly Ser Val Leu Gly Arg
           35                  40                  45
   Gly His Asn Met Arg Phe Gln Lys Gly Ser Ala Thr Leu His Gly Glu
       50                  55                  60
   Ile Ser Thr Leu Glu Asn Cys Gly Arg Leu Glu Gly Lys Val Tyr Lys
   65                  70                  75                  80
   Asp Thr Thr Leu Tyr Thr Thr Leu Ser Pro Cys Asp Met Cys Thr Gly
                   85                  90                  95
   Ala Ile Ile Met Tyr Gly Ile Pro Arg Cys Val Val Gly Glu Asn Val
               100                 105                 110
   Asn Phe Lys Ser Lys Gly Glu Lys Tyr Leu Gln Thr Arg Gly His Glu
           115                 120                 125
   Val Val Val Val Asp Asp Glu Arg Cys Lys Lys Ile Met Lys Gln Phe
       130                 135                 140
   Ile Asp Glu Arg Pro Gln Asp Trp Phe Glu Asp Ile Gly Glu Ala Ser
   145                 150                 155                 160
   Glu Pro Phe Lys Asn Val Tyr Leu Leu Pro Gln Thr Asn Gln Leu Leu
                   165                 170                 175
   Gly Leu Tyr Thr Ile Ile Ser Asn Lys Asn Thr Thr Arg Pro Asp Phe
               180                 185                 190
   Ile Phe Tyr Ser Asp Arg Ile Ile Arg Leu Leu Val Glu Glu Gly Leu
           195                 200                 205
   Asn His Leu Pro Val Gln Lys Gln Ile Val Glu Thr Asp Thr Asn Glu
       210                 215                 220
   Asn Phe Glu Gly Val Ser Phe Met Gly Lys Ile Cys Gly Val Ser Ile
   225                 230                 235                 240
   Val Arg Ala Gly Glu Ser Met Glu Gln Gly Leu Arg Asp Cys Cys Arg
                   245                 250                 255
   Ser Val Arg Ile Gly Lys Ile Leu Ile Gln Arg Asp Glu Glu Thr Ala
               260                 265                 270
   Leu Pro Lys Leu Phe Tyr Glu Lys Leu Pro Glu Asp Ile Ser Glu Arg
           275                 280                 285
   Tyr Val Phe Leu Leu Asp Pro Met Leu Ala Thr Gly Gly Ser Ala Ile
       290                 295                 300
   Met Ala Thr Glu Val Leu Ile Lys Arg Gly Val Lys Pro Glu Arg Ile
   305                 310                 315                 320
   Tyr Phe Leu Asn Leu Ile Cys Ser Lys Glu Gly Ile Glu Lys Tyr His
                   325                 330                 335
   Ala Ala Phe Pro Glu Val Arg Ile Val Thr Gly Ala Leu Asp Arg Gly
               340                 345                 350
   Leu Asp Glu Asn Lys Tyr Leu Val Pro Gly Leu Gly Asp Phe Gly Asp
           355                 360                 365
   Arg Tyr Tyr Cys Val
       370
<210> 3
   <211> 644
   <212> PRT
   <213> Artificial Sequence
<220>
   <223> derived from Escherichia Coli
<400> 3
   Met Val Ser Asn Asn Ala Leu Glu Thr Ile Ile Asn Ala Arg Leu Pro
   1               5                   10                  15
   Gly Glu Glu Gly Leu Trp Gln Ile His Leu Gln Asp Gly Lys Ile Ser
               20                  25                  30
   Ala Ile Asp Ala Gln Ser Gly Val Met Pro Ile Thr Glu Asn Ser Leu
           35                  40                  45
   Asp Ala Glu Gln Gly Leu Val Ile Pro Pro Phe Val Glu Pro His Ile
       50                  55                  60
   His Leu Asp Thr Thr Gln Thr Ala Gly Gln Pro Asn Trp Asn Gln Ser
   65                  70                  75                  80
   Gly Thr Leu Phe Glu Gly Ile Glu Arg Trp Ala Glu Arg Lys Ala Leu
                   85                  90                  95
   Leu Thr His Asp Asp Val Lys Gln Arg Ala Trp Gln Thr Leu Lys Trp
               100                 105                 110
   Gln Ile Ala Asn Gly Ile Gln His Val Arg Thr His Val Asp Val Ser
           115                 120                 125
   Asp Ala Thr Leu Thr Ala Leu Lys Ala Met Leu Glu Val Lys Gln Glu
       130                 135                 140
   Val Ala Pro Trp Ile Asp Leu Gln Ile Val Ala Phe Pro Gln Glu Gly
   145                 150                 155                 160
   Ile Leu Ser Tyr Pro Asn Gly Glu Ala Leu Leu Glu Glu Ala Leu Arg
                   165                 170                 175
   Leu Gly Ala Asp Val Val Gly Ala Ile Pro His Phe Glu Phe Thr Arg
               180                 185                 190
   Glu Tyr Gly Val Glu Ser Leu His Lys Thr Phe Ala Leu Ala Gln Lys
           195                 200                 205
   Tyr Asp Arg Leu Ile Asp Val His Cys Asp Glu Ile Asp Asp Glu Gln
       210                 215                 220
   Ser Arg Phe Val Glu Thr Val Ala Ala Leu Ala His His Glu Gly Met
   225                 230                 235                 240
   Gly Ala Arg Val Thr Ala Ser His Thr Thr Ala Met His Ser Tyr Asn
                   245                 250                 255
   Gly Ala Tyr Thr Ser Arg Leu Phe Arg Leu Leu Lys Met Ser Gly Ile
               260                 265                 270
   Asn Phe Val Ala Asn Pro Leu Val Asn Ile His Leu Gln Gly Arg Phe
           275                 280                 285
   Asp Thr Tyr Pro Lys Arg Arg Gly Ile Thr Arg Val Lys Glu Met Leu
       290                 295                 300
   Glu Ser Gly Ile Asn Val Cys Phe Gly His Asp Asp Val Phe Asp Pro
   305                 310                 315                 320
   Trp Tyr Pro Leu Gly Thr Ala Asn Met Leu Gln Val Leu His Met Gly
                   325                 330                 335
   Leu His Val Cys Gln Leu Met Gly Tyr Gly Gln Ile Asn Asp Gly Leu
               340                 345                 350
   Asn Leu Ile Thr His His Ser Ala Arg Thr Leu Asn Leu Gln Asp Tyr
           355                 360                 365
   Gly Ile Ala Ala Gly Asn Ser Ala Asn Leu Ile Ile Leu Pro Ala Glu
       370                 375                 380
   Asn Gly Phe Asp Ala Leu Arg Arg Gln Val Pro Val Arg Tyr Ser Val
   385                 390                 395                 400
   Arg Gly Gly Lys Val Ile Ala Ser Thr Gln Pro Ala Gln Thr Thr Val
                   405                 410                 415
   Tyr Leu Glu Gln Pro Glu Ala Ile Asp Tyr Lys Arg Ile Ser Gly Ala
               420                 425                 430
   Asn Gly Val Met Ala Lys Ile Val Glu Val Lys His Pro Leu Val Lys
           435                 440                 445
   His Lys Leu Gly Leu Met Arg Glu Gln Asp Ile Ser Thr Lys Arg Phe
       450                 455                 460
   Arg Glu Leu Ala Ser Glu Val Gly Ser Leu Leu Thr Tyr Glu Ala Thr
   465                 470                 475                 480
   Ala Asp Leu Glu Thr Glu Lys Val Thr Ile Glu Gly Trp Asn Gly Pro
                   485                 490                 495
   Val Glu Ile Asp Gln Ile Lys Gly Lys Lys Ile Thr Val Val Pro Ile
               500                 505                 510
   Leu Arg Ala Gly Leu Gly Met Met Asp Gly Val Leu Glu Asn Val Pro
           515                 520                 525
   Ser Ala Arg Ile Ser Val Val Gly Met Tyr Arg Asn Glu Glu Thr Leu
       530                 535                 540
   Glu Pro Val Pro Tyr Phe Gln Lys Leu Val Ser Asn Ile Asp Glu Arg
   545                 550                 555                 560
   Met Ala Leu Ile Val Asp Pro Met Leu Ala Thr Gly Gly Ser Val Ile
                   565                 570                 575
   Ala Thr Ile Asp Leu Leu Lys Lys Ala Gly Cys Ser Ser Ile Lys Val
               580                 585                 590
   Leu Val Leu Val Ala Ala Pro Glu Gly Ile Ala Ala Leu Glu Lys Ala
           595                 600                 605
   His Pro Asp Val Glu Leu Tyr Thr Ala Ser Ile Asp Gln Gly Leu Asn
       610                 615                 620
   Glu His Gly Tyr Ile Ile Pro Gly Leu Gly Asp Ala Gly Asp Lys Ile
   625                 630                 635                 640
   Phe Gly Thr Lys
<210> 4
   <211> 158
   <212> PRT
   <213> Saccharomyces cerevisiae
<400> 4
   Met Val Thr Gly Gly Met Ala Ser Lys Trp Asp Gln Lys Gly Met Asp
   1               5                   10                  15
   Ile Ala Tyr Glu Glu Ala Ala Leu Gly Tyr Lys Glu Gly Gly Val Pro
               20                  25                  30
   Ile Gly Gly Cys Leu Ile Asn Asn Lys Asp Gly Ser Val Leu Gly Arg
           35                  40                  45
   Gly His Asn Met Arg Phe Gln Lys Gly Ser Ala Thr Leu His Gly Glu
       50                  55                  60
   Ile Ser Thr Leu Glu Asn Cys Gly Arg Leu Glu Gly Lys Val Tyr Lys
   65                  70                  75                  80
   Asp Thr Thr Leu Tyr Thr Thr Leu Ser Pro Cys Asp Met Cys Thr Gly
                   85                  90                  95
   Ala Ile Ile Met Tyr Gly Ile Pro Arg Cys Val Val Gly Glu Asn Val
               100                 105                 110
   Asn Phe Lys Ser Lys Gly Glu Lys Tyr Leu Gln Thr Arg Gly His Glu
           115                 120                 125
   Val Val Val Val Asp Asp Glu Arg Cys Lys Lys Ile Met Lys Gln Phe
       130                 135                 140
   Ile Asp Glu Arg Pro Gln Asp Trp Phe Glu Asp Ile Gly Glu
   145                 150                 155
<211> 150
   <212> PRT
   <213> Candida albicans
<400> 5
<210> 6
   <211> 432
   <212> PRT
   <213> Escherichia coli
<400> 6
   Met Trp Arg Leu Thr Val Ser Asn Asn Ala Leu Gln Thr Ile Ile Asn
   1               5                   10                  15
   Ala Gln Leu Pro Gly Lys Glu Gly Leu Trp Gln Ile His Leu His Asp
               20                  25                  30
   Gly Lys Ile Ser Ala Ile Asp Ala Gln Ser Gly Val Met Pro Val Thr
           35                  40                  45
   Glu Asn Ser Leu Asp Ala Glu Gln Gly Leu Val Leu Pro Pro Phe Val
       50                  55                  60
   Glu Pro His Ile His Leu Asp Thr Thr Gln Thr Ala Gly Gln Pro Asn
   65                  70                  75                  80
   Trp Asn Gln Ser Gly Thr Leu Phe Glu Gly Ile Glu Arg Trp Ala Glu
                   85                  90                  95
   Arg Lys Ala Leu Leu Thr His Asp Asp Val Lys Gln Arg Ala Trp Gln
               100                 105                 110
   Thr Leu Lys Trp Gln Ile Ala Asn Gly Ile Gln His Val Arg Thr His
           115                 120                 125
   Val Asp Val Ser Asp Ala Thr Leu Thr Ala Leu Lys Ala Met Leu Glu
       130                 135                 140
   Val Lys Gln Glu Val Ala Pro Trp Ile Asp Leu Gln Ile Val Ala Phe
   145                 150                 155                 160
   Pro Gln Glu Gly Ile Leu Ser Tyr Pro Asn Gly Glu Ala Leu Leu Glu
                   165                 170                 175
   Glu Ala Leu Arg Leu Gly Ala Asp Val Val Gly Ala Ile Pro His Phe
               180                 185                 190
   Glu Phe Thr Arg Glu Tyr Gly Val Glu Ser Leu His Lys Thr Phe Ala
           195                 200                 205
   Leu Ala Gln Lys Tyr Asp Arg Leu Ile Asp Val His Cys Asp Glu Ile
       210                 215                 220
   Asp Asp Glu Gln Ser Arg Phe Val Glu Thr Val Ala Ala Leu Ala His
   225                 230                 235                 240
   Arg Glu Gly Met Gly Ala Arg Val Thr Ala Ser His Thr Thr Ala Met
                   245                 250                 255
   His Ser Tyr Asn Gly Ala Tyr Thr Ser Arg Leu Phe Arg Leu Leu Lys
               260                 265                 270
   Met Ser Gly Ile Asn Phe Val Ala Asn Pro Leu Val Asn Ile His Leu
           275                 280                 285
   Gln Gly Arg Phe Asp Thr Tyr Pro Lys Arg Arg Gly Ile Thr Arg Val
       290                 295                 300
   Lys Glu Met Leu Glu Ser Gly Ile Asn Val Cys Phe Gly His Asp Asp
   305                 310                 315                 320
   Val Phe Asp Pro Trp Tyr Pro Leu Gly Thr Ala Asn Met Leu Gln Val
                   325                 330                 335
   Leu His Met Gly Leu His Val Cys Gln Leu Met Gly Tyr Gly Gln Ile
               340                 345                 350
   Asn Asp Gly Leu Asn Leu Ile Thr His His Ser Ala Arg Thr Leu Asn
           355                 360                 365
   Leu Gln Asp Tyr Gly Ile Ala Ala Gly Asn Ser Ala Asn Leu Ile Ile
       370                 375                 380
   Leu Pro Ala Glu Asn Gly Phe Asp Ala Leu Arg Arg Gln Val Pro Val
   385                 390                 395                 400
   Arg Tyr Ser Val Arg Gly Gly Lys Val Ile Ala Ser Thr Gln Pro Ala
                   405                 410                 415
   Gln Thr Thr Val Tyr Leu Glu Gln Pro Glu Ala Ile Asp Tyr Lys Arg
               420                 425                 430
<210> 7
   <211> 30
   <212> DNA
   <213> Artificial Sequence
<220>
   <221> source
   <222> 1..30
   <223> /mol_type="unassigned DNA" /note="oligonucleotide" /organism="Artificial Sequence"
<400> 7
   tcccccgggt taaccactgc atgatgtaca 30
<210> 8
   <211> 30
   <212> DNA
   <213> Artificial Sequence
<220>
   <221> source
   <222> 1..30
   <223> /mol_type="unassigned DNA" /note="oligonucleotides" /organism="Artificial Sequence"
<400> 8
   gccgagctcg aggtagccgt ttgtaattct 30
<210> 9
   <211> 24
   <212> DNA
   <213> Artificial Sequence
<220>
   <221> source
   <222> 1..24
   <223> /mol_type="unassigned DNA" /note="oligonucleotides" /organism="Artificial Sequence"
<400> 9
   gcctggccat aactccaggc cgtt 24
<210> 10
   <211> 30
   <212> DNA
   <213> Artificial Sequence
<220>
   <221> source
   <222> 1..30
   <223> /mol_type="unassigned DNA" /note="oligonucleotides" /organism="Artificial Sequence"
<400> 10
   gcccagctga tcgagccgta acgattttca 30
<210> 11
   <211> 30
   <212> DNA
   <213> Artificial Sequence
<220>
   <221> source
   <222> 1..30
   <223> /mol_type="unassigned DNA" /note="oligonucleotides" /organism="Artificial Sequence"
<400> 11
   gccgcatgca tccttgaaca ccaataccga 30
<210> 12
   <211> 29
   <212> DNA
   <213> Artificial Sequence
<220>
   <221> source
   <222> 1..29
   <223> /mol_type="unassigned DNA" /note="oligonucleotide" /organism="Artificial Sequence"
<400> 12
   gctctagaga ggtagccgtt tgtaatctg 29
<210> 13
   <211> 30
   <212> DNA
   <213> Artificial Sequence
<220>
   <221> source
   <222> 1..30
   <223> /mol_type="unassigned DNA" /note="oligonucleotide" /organism="Artificial Sequence"
<400> 13
   cgcggatcct ttggtacagt ctagtatcca 30
<210> 14
   <211> 30
   <212> DNA
   <213> Artificial Sequence
<220>
   <221> source
   <222> 1..30
   <223> /mol_type="unassigned DNA" /note="oligonucleotide" /organism="Artificial Sequence"
<400> 14
   tcccccgggt tataacagat gcagtatcca 30
<210> 15
   <211> 30
   <212> DNA
   <213> Artificial Sequence
<220>
   <221> source
   <222> 1..30
   <223> /mol_type="unassigned DNA" /note="oligonucleotide" /organism="Artificial Sequence"
<400> 15
   gcccagctgt tcaatggcca tctgaaatcc 30
<210> 16
   <211> 29
   <212> DNA
   <213> Artificial Sequence
<220>
   <221> source
   <222> 1..29
   <223> /mol_type="unassigned DNA" /note="oligonucleotide" /organism="Artificial Sequence"
<400> 16
   gaagatctag tatcgcatct aaaagatgg 29
<210> 17
   <211> 30
   <212> DNA
   <213> Artificial Sequence
<220>
   <221> source
   <222> 1..30
   <223> /mol_type="unassigned DNA" /note="oligonucleotide" /organism="Artificial Sequence"
<400> 17
   gccgagctca cccacacgtt tttcgaaaaa 30
<210> 18
   <211> 30
   <212> DNA
   <213> Artificial Sequence
<220>
   <221> source
   <222> 1..30
   <223> /mol_type="unassigned DNA" /note="oligonucleotide" /organism="Artificial Sequence"
<400> 18
   gccgcatgct tataacagat gcagtatcaa 30

## Description

### Technical Field

Oncolytic viruses are a class of novel therapeutic agents used for the treatment of cancer that have the unique property of tumor-dependent self-perpetuation (HERMISTON. A demand for next-generation oncolytic adenoviruses. Current opinion in molecular therapeutics. 2006, vol.8, no.4, p.322-30.). Oncolytic viruses are capable of selective replication in malignant cells and therefore offer levels of potency and specificity that are potentially far higher than conventional treatments for cancer (FISHER. Striking out at disseminated metastases: the systemic delivery of oncolytic viruses. Current opinion in molecular therapeutics. 2006, vol.8, no.4, p.301-13.). The benefit of using these viruses is that as they replicate, they lyse their host cells. Cancer cells are ideal hosts for many viruses because they have the antiviral interferon pathway inactivated or have mutated tumour suppressor genes that enable viral replication to proceed unhindered (CHERNAJOVSKY, et al. Fighting cancer with oncolytic viruses. British medical journal. 2006, vol.332, no.7534, p.170-2.).

Some viruses are naturally able to selectively replicate in tumoral cells but oncolytic viruses can also be obtained by modifying naturally occurring viruses. For this purpose, the main strategies used currently to modify the viruses include: functional deletions in essential viral genes; tumor- or tissue-specific promoters used to control the expression of these viral genes; and tropism modification to redirect adenovirus to the cancer cell surface. In the near future, oncolytic adenoviruses need to be optimized to fully realize their potential as critical anticancer tools and, thus, improve the prognosis for patients with malignant gliomas (JIANG, et al. Oncolytic adenoviruses as antiglioma agents. Expert review of anticancer therapy. 2006, vol.6, no.5, p.697-708.).

For example, ONYX-015, an adenovirus modified selectively to replicate in and kill cells that harbor p53 mutations, is under development by Onyx Pharmaceuticals for the potential treatment of various solid tumors, including head and neck, gastrointestinal and pancreatic tumors. It is a recombinant adenovirus that carries a loss-of-function mutation at the E1B locus, the product of which is a 55 kDa protein that binds to and inactivates the p53 tumor suppressor protein. Thus, the ONYX-015 adenovirus is supposed to leave normal cells unaffected. Mutations in the p53 tumor suppressor gene are the most common type of genetic abnormality in cancer, occurring in more than half of all major cancer types. Thus, these cells are susceptible to the virus, which will readily replicate and cause cell death. ONYX-015 is in ongoing phase III trials for the treatment of recurrent head and neck cancer, phase II trials for colorectal, ovary, pancreas and mouth tumors, and phase I trials for digestive disease, esophagus and liver tumors (COHEN, et al. ONYX-015. Onyx Pharmaceuticals. Current opinion in investigational drugs. 2001, vol.2, no.12, p.1770-5.).

Naturally oncolytic viruses are replication-competent viruses that have an innate ability to selectively infect and kill tumor cells. Despite being used in the original attempts to treat cancer with live viruses five decades ago, interest in naturally oncolytic viruses has lagged behind the support for engineered adenoviruses and herpesviruses as cancer therapeutics. Recently, however, there has been renewed interest in the high potency and selectivity of these naturally occurring agents (ROBERTS, et al. Naturally oncolytic viruses. Current opinion in molecular therapeutics. 2006, vol.8, no.4, p.314-21.).

Among naturally oncolytic viruses, Vaccinia viruses (a Poxviridae) possess many of the key attributes necessary for an ideal viral backbone for use in oncolytic virotherapy. These include a short lifecycle, with rapid cell-to-cell spread. strong lytic ability, a large cloning capacity and well-defined molecular biology. In addition, although capable of replicating in human cells, they are not considered a natural health problem and are especially well characterized having been delivered to millions of individuals during the campaign to eradicate smallpox. Early clinical results using either vaccine strains or genetically modified vaccinia strains have demonstrated antitumor effects (THORNE, et al. Vaccinia virus and oncolytic virotherapy of cancer. Current opinion in molecular therapeutics. 2005, vol.7, no.4, p.359-65.).

In contrast, the poxvirus myxoma virus is a novel oncolytic candidate that has no history of use in humans directly, as it has a distinct and absolute host species tropism to lagomorphs (rabbits). Myxoma virus has been recently shown to be able to also selectively infect and kill human tumor cells, a unique tropism that is linked to dysregulated intracellular signalling pathways found in the majority of human cancers. This review outlines the existing knowledge on the tropism of myxoma virus for human cancer cells, as well as preclinical data exhibiting its ability to infect and clear tumors in animal models of cancer (STANFORD, et al. Myxoma virus and oncolytic virotherapy: a new biologic weapon in the war against cancer. Expert opinion on biological therapy. 2007, vol.7, no.9, p.1415-25.).

### Technical Problem

The injection of high doses of Poxviruses necessary to achieve an antitumoral effect raised toxicity issues. The majority of adverse events are minor, adverse reactions that are usually linked to Vaccinia virus are self-limited and include fever, headache, fatigue, myalgia, chills, local skin reactions, nonspecific rashes, erythema multiforme, lymphadenopathy, and pain at the vaccination site. Other reactions might require additional therapies (e.g., VIG, a first-line therapy and cidofovir, a second-line therapy). Adverse reactions that might require further evaluation or therapy include inadvertent inoculation, generalized vaccinia (GV), eczema vaccinatum (EV), progressive vaccinia (PV), postvaccinial central nervous system disease, and fetal vaccinia (CONO, et al. Smallpox vaccination and adverse reactions. Guidance for clinicians. MMWR. Recommendations and reports : Morbidity and mortality weekly report. Recommendations and reports / Centers for Disease Control. 2003, vol.52, no.RR-4, p.1-28.).

Thus, there is need for safer Poxviruses with an oncolytic activity as good as to their natural counterparts.

### Background Art

US 5364773 (VIROGENETICS CORPORATION (TROY, NY)) 15/11/1994 describes a modified recombinant poxvirus, more particularly a vaccinia virus having inactivated nonessential virus-encoded encoded genetic functions so that the recombinant poxvirus has attenuated virulence and enhanced safety. In particular, the genetic functions are inactivated by deleting an open reading frame encoding a virulence factor or by insertional inactivation of an open reading frame encoding a virulence factor. More particularly, this patent describes a vaccinia virus in which the open reading frame of for J2R, B13R+B14R, A26L, A56R, C7L - K1L, and I4L has been inactivated. This virus (NYVAC) can be engineered as a vector for a foreign nucleic acid and used as a vaccine for inducing an immunological response in a host animal. However, N YVAC is unable to efficiently replicate in most mammalian cels and can not be used as an oncolytic virus (XIANGZHI, et al. Vaccinia virus K1L protein supports viral replication in human and rabbit cells through a cell-type-specific set of its ankyrin repeat residues that are distinct from its binding site for ACAP2. Journal of virology. 2006, vol.353, no.1, p.220-233.).

WO 2004/014314 (KIRN DAVID (US)) 19/02/2004 describes an altered vaccinia virus that comprises one or more mutations in its viral genome. Described mutations are in one or more of the following classes of polypeptides: 1) interferon-modulating polypeptide; 2) complement control polypeptide ; 3) TNF or chemokine-modulating polypeptide; 4) serine protease inhibitor; 5) IL- Ip modulating polypeptide; 6) non-infectious EEV form polypeptides; and, 7) viral polypeptide that act to inhibit release of infectious virus from cells (anti-infectious virus form polypeptide). In addition, mutations in A41L or C11R of vaccinia virus are also disclosed.

### Disclosure of Invention

The present invention relates to
a composition comprising an oncolytic poxvirus comprising a defective gene encoding the large subunit of ribonucleotide reductase (I4L) and/or the small subunit of ribonucleotide reductase (F4L) and a defective gene encoding thymidine kinase (J2R) and further comprising a suicide gene for use in treating cancer in combination with one or more substances effective in anticancer therapy, wherein the one or more substances effective in anticancer therapy are selected from irinotecan and oxaliplatin.

As used throughout the entire application, the terms "a" and "an" are used in the sense that they mean "at least one", "at least a first", "one or more" or "a plurality" of the referenced components or steps, unless the context clearly dictates otherwise. For example, the term "a cell" includes a plurality of cells, including mixtures thereof.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range.

As used herein, the terms "comprising" and "comprise" are intended to mean that the products, compositions and methods include the referenced components or steps, but not excluding others. "Consisting essentially of" when used to define products, compositions and methods, shall mean excluding other components or steps of any essential significance. Thus, a composition consisting essentially of the recited components would not exclude trace contaminants and pharmaceutically acceptable carriers. "Consisting of' shall mean excluding more than trace elements of other components or steps.

As used herein, the term "poxvirus comprising a defective gene" refers to a poxvirus comprising a deletion, substitution or addition in one or more nucleic acid of the defective gene, or any combination of these possibilities wherein said modifications lead to the inability for the virus to produce a protein having the activity of the protein produced by the unmodified gene. In a preferred embodiment of the invention, a poxvirus comprising a defective gene refers to a poxvirus in which the whole gene sequence has been deleted. Mutation can be made in a number of ways known to those skilled in the art using recombinant techniques. Methods for modifying the genome of a poxvirus are available in the art. For example the methods disclosed in MCCART, et al. Systemic cancer therapy with a tumor selective vaccinia virus mutant lacking thymidine kinase and vaccinia growth factor genes.. Cancer res.. 2001, no.61, p.8751-57., KIM, et al. Systemic armed oncolytic ans immunologic therapy for cancer with JX-594, a targeted poxvirus expressing GM-CSF. Molecular Therapeutic. 2006, no.14, p.361-70., WO 2004/014314 (KIRN DAVID (US)) 19/02/2004 and US 5364773 (VIROGENETICS CORPORATION (TROY, NY)) 15/11/1994 can be used to produce the poxvirus for use according to the invention. The methods disclosed in the example of the present application are particularly relevant to produce a poxvirus for use according to according to the invention.
Sequences of the genome of various poxviruses are available in the art, for example, the vaccinia virus, cowpox virus, Canarypox virus, Ectromelia virus, Myxoma virus genomes are available in Genbank (accession number NC_006998, NC_003663, NC_005309, NC_004105, NC_001132 respectively)

As used herein the term "poxvirus" refers to a virus belonging to the Poxviridae family. According to a preferred embodiment, the poxvirus according to the invention belongs to the Chordopoxvirinae subfamily, more preferably to the Orthopoxvirus genus and even more preferably to the Vaccinia virus specie.

For example, Vaccinia virus strains Dairen I, IHD-J, L-IPV, LC16M8, LC16MO, Lister, LIVP, Tashkent, WR 65-16, Wyeth, Ankara, Copenhagen, Tian Tan and WR can be used. According to a particularly preferred embodiment, the poxvirus for use according to the invention is a Vaccinia virus strains Copenhagen.

The poxvirus vaccinia contains a large duplex DNA genome (187 kilobase pairs) and is a member of the only known family of DNA viruses that replicates in the cytoplasm of infected cells. Because the infected cell must deliver large amounts of DNA precursors to cytoplasmic replication sites, the virus encodes and expresses many enzymatic activities required for DNA metabolism and synthesis, including ribonucleotide reductase and deoxyuridine 5'-triphosphate nucleotidohydrolase (dUTPase).

Ribonucleotide reductase (EC 1.17.4.1) catalyzes the reduction of ribonucleotides to deoxyribonucleotides, a reaction that represents the first commited step in DNA biosynthesis. The viral enzyme is similar in subunit structure to the mammalian enzyme, being composed of two heterologous subunits, designed R1 and R2. The genes that encode the viral ribonucleotide reductase subunits have been sequenced and localized to positions on the vaccinia genome, separated by 35 kilobases (SLABAUGH, et al.. Journal of virology. 1988, vol.62, p.519-27.; TENGELSEN, etal.. Virology. 1988, no.164, p.121-31. ; SCHMITT, et al.. Journal of virology. 1988, no.62, p.1889-97.). The monomers of the vaccinia virus large subunit (designated R1, encoded by the I4L gene) are 86-kDa polypeptides, and contain binding sites for nucleotide substrates an allosteric effectors (SLABAUGH, et al.. Journal of virology. 1984, no.52, p.507-14. ; SLABAUGH, et al.. Journal of virology. 1984, no.52, p.501-6.). The small subunit (designated R2, encoded by the F4L gene) is a homodimer comprising two 37-kDa polypeptides; each polypeptide contains an iron-stabilized protein-based free radical that is required for catalysis (HOWELL, et al.. Journal of Biological Chemistry. 1992, no.267, p.1705-11.). Sequences for the I4L and F4L genes and their locations in the genome of various poxvirus are available in public databases, for example via accession number DQ437594, DQ437593, DQ377804, AH015635, AY313847, AY313848, NC_003391, NC_003389, NC_003310, M35027, AY243312, DQ011157, DQ011156, DQ011155, DQ011154, DQ011153, Y16780, X71982, AF438165, U60315, AF410153, AF380138, U86916, L22579, NC_006998, DQ121394 and NC_008291.

The gene nomenclature used herein is that of Copenhagen vaccinia strain and is used also for the homologous genes of other poxviridae unless otherwise indicated. However, gene nomenclature may be different according to the pox strain. For information, correspondance between Copenhagen and MVA genes can be found in Table I of ANTOINE. Virology. 1998, no.244, p.365-396.

The J2R gene encodes a Thymidine kinase (TK) which form part of the salvage pathway for pyrimidine deoxyribonucleotide synthesis. The reaction catalysed by TK involves the transfer of a γ-phosphoryl moiety from ATP to 2'deoxy-thymidine (dThd) to produce thymidine 5'-monophosphate (dTMP). Vaccinia virus' TK is of type 2. Type 2 TKs have a smaller polypeptide chain compared to type 1, being of -25 KDa but form homotetramers. They are sensitive to the feedback inhibitors dTDP or dTTP, which are generated at the end of the metabolic pathway. Type 2 TKs have a much narrower substrate specificity compared to type 1 TKs and only phosphorylate 2'deoxyuridine (dU) and/or dThd (EL OMARI, et al. Structure of vaccinia virus thymidine kinase in complex with dTTP: insights for drug design. BMC structural biology. 2006, no.6, p.22.).

Poxviruses defective for the J2R region and methods to obtain them are available in the art. For example, the teaching of MCCART, et al. Systemic cancer therapy with a tumor-selective vaccinia virus mutant lacking thymidine kinase and vaccinia growth factor genes. cancer research. 2001, vol.61, no.24, p.8751-7., PUHLMANN, et al. Vaccinia as a vector for tumor-directed gene therapy: biodistribution of a thymidine kinase-deleted mutant. Cancer gene therapy. 2000, vol.7, no.1, p.66-73. , GNANT, et al. Systemic administration of a recombinant vaccinia virus expressing the cytosine deaminase gene and subsequent treatment with 5-fluorocytosine leads to tumor-specific gene expression and prolongation of survival in mice. Cancer Research. 1999, vol.59, no.14, p.3396-403. can be used to produced a poxviruses deleted for the J2R region.

According to a preferred embodiment, the poxvirus for use according to the invention further comprises a defective F2L gene.

Deoxyuridine 5'-triphosphate nucleotidohydrolase (dUTPase, EC 3.6.1.23) catalyzes the hydrolysis of dUTP to dUMP and pyrophosphate in the presence of Mg(2+) ions. dUTPase, in removing dUTP from the dNTP pool and generating dUMP, is involved in both maintaining the fidelity of DNA replication and in proviging the precursor for the production of TMP by thymidylate synthase. Vaccinia dUTPase is a 15 kDa protein encoded by the F2L gene (MCGEOGH.. Nucleic Acids Research. 1990, no.18, p.4105-10. ; BROYLES.. Virology. 1993, no.195, p.863-5.). Sequence of the F2L gene of the vaccinia virus is available in genbank via accession number M25392, sequences and locations of the F2L gene in various poxviruses genomes are also available in genbank, for example, via accession number NC_006998, DQ121394, NC_001611, AY689436, AY689437, NC_008291, DQ437594, DQ437593, AY313847, AY313848, NC_006966, NC_005309, NC_003391, NC_003389, NC_001132, NC_003310, NC_002188, M35027, AY243312, AF170726, DQ011157, DQ011156, DQ011155, DQ011154, DQ011153, X94355, Y16780, AY318871, U94848, AF198100 and M34368.

According to a preferred aspect, the poxvirus may further comprise a nucleic acid of interest.

The nucleic acid of interest may contain at least one sequence of interest encoding a gene product which is a therapeutic molecule (i.e. a therapeutic gene). A "therapeutic molecule" is one which has a pharmacological or protective activity when administered appropriately to a patient, especially patient suffering from a disease or illness condition or who should be protected against this disease or condition. Such a pharmacological or protective activity is one which is expected to be related to a beneficial effect on the course or a symptom of said disease or said condition. When the skilled man selects in the course of the present invention a gene encoding a therapeutic molecule, he generally relates his choice to results previously obtained and can reasonably expect, without undue experiment other than practicing the invention as claimed, to obtain such pharmacological property. The sequence of interest can be homologous or heterologous to the target cells into which it is introduced. Advantageously said sequence of interest encodes all or part of a polypeptide, especially a therapeutic or prophylactic polypeptide giving a therapeutic or prophylactic property. A polypeptide is understood to be any translational product of a polynucleotide regardless of size, and whether glycosylated or not, and includes peptides and proteins. Therapeutic polypeptides include as a primary example those polypeptides that can compensate for defective or deficient proteins in an animal or human organism, or those that act through toxic effects to limit or remove harmful cells from the body. They can also be immunity conferring polypeptides which act as endogenous antigen to provoke a humoral or cellular response, or both.

Examples of polypeptides encoded by a therapeutic gene include genes coding for a cytokine (alpha, beta or gamma interferon, interleukin, in particular IL-2, IL-6, IL-10 or IL-12, a tumor necrosis factor (TNF), a colony stimulating factor GM-CSF, C-CSF, M-CSF...), a immunostimulatory polypeptide (B7.1, B7.2 and the like), a coagulation factor (FVIII, FIX...), a growth factor (Transforming Growth Factor TGF, Fibroblast Growth Factor FGF and the like), an enzyme (urease, renin, thrombin, metalloproteinase, nitric oxide synthase NOS, SOD, catalase...), an enzyme inhibitor (alpha1-antitrypsin, antithrombin III, viral protease inhibitor, plasminogen activator inhibitor PAI-1), the CFTR (Cystic Fibrosis Transmembrane Conductance Regulator) protein, insulin, dystrophin, a MHC antigen of class I or II, a polypeptide that can modulate/regulate expression of cellular genes, a polypeptide capable of inhibiting a bacterial, parasitic or viral infection or its development (antigenic polypeptides, antigenic epitopes, transdominant variants inhibiting the action of a native protein by competition....), an apoptosis inducer or inhibitor (Bax, Bcl2, BclX...), a cytostatic agent (p21, p 16, Rb...), an apolipoprotein (ApoAI, ApoAIV, ApoE...), an inhibitor of angiogenesis (angiostatin, endostatin...), an angiogenic polypeptide (family of Vascular Endothelial Growth Factors VEGF, FGF family, CCN family including CTGF, Cyr61 and Nov), an oxygen radical scaveyer, a polypeptide having an anti-tumor effect, an antibody, a toxin, an immunotoxin and a marker (beta-galactosidase, luciferase....) or any other genes of interest that are recognized in the art as being useful for the treatment or prevention of a clinical condition.

Suitable anti-tumor genes include but are not limited to those encoding tumor suppressor genes (e.g. Rb, p53, DCC, NF-1, Wilm's tumor, NM23, BRUSH-1, p16, p21, p56, p73 as well as their repective mutants), suicide gene products, antibodies, polypeptides inhibiting cellular division or transduction signals.

Suicide gene refers to a gene coding a protein able to convert a precursor of a drug into a cytoxic compound.

Suicide genes comprise but are not limited to genes coding protein having a cytosine deaminase activity, a thymidine kinase activity, an uracil phosphoribosyl transferase activity, a purine nucleoside phosphorylase activity and/or a thymidylate kinase activity.

Examples of suicide genes and corresponding precursors of a drug comprising one nucleobase moiety are disclosed in the following table :

**Table 1**

| Suicide gene | predrug |
|---|---|
| Thymidine Kinase | Ganciclovir; |
| | Ganciclovir elaidic acid ester; |
| | penciclovir; |
| | Acyclovir; |
| | Valacyclovir; |
| | (E)-5-(2-bromovinyl)-2'-deoxyuridine; |
| | zidovudine; |
| | 2'-Exo-methanocarbathymidine |
| Cytosine deaminase | 5-Fluorocytosine |
| Purine nucleoside phosphorylase | 6-Methylpurine deoxyriboside; Fludarabine |
| uracil phosphoribosyl transferase | 5-Fluorocytosine; |
| | 5-Fluorouracil |
| thymidylate kinase. | Azidothymidine |

According to a preferred embodiment of the invention, the suicide gene codes a protein having at least a CDase activity. CDase is involved in the pyrimidine metabolic pathway by which exogenous cytosine is transformed into uracil by means of a hydrolytic deamination. While CDase activities have been demonstrated in prokaryotes and lower eukaryotes (JUND, et al.. Journal of Bacteriology. 1970, no. 102, p.607-15. ; BECK, et al.. Journal of Bacteriology. 1972, no.110, p.219-28. ; HOEPRICH, et al.. Journal of Infectious Diseases. 1974, no. 130, p.112-18. ; ESDERS, et al.. J. biol. chem.. 1985, no.260, p.3915-22.), they are not present in mammals (KOECHLIN, et al.. Biochemical pharmacology. 1966, no.15, p.435-46. ; POLAK, et al.. Chemotherapy. 1976, no.22, p.137-53.).

CDase also deaminates an analogue of cytosine, i.e. 5-fluorocytosine (5-FC), thereby forming 5-fluorouracil (5-FU), which is a compound which is highly cytotoxic when it is converted into 5-fluoro-UMP (5-FUMP). Cells which lack CDase activity, either because of a mutation which inactivates the gene encoding the enzyme or because they are naturally deficient in this enzyme, as are mammalian cells, are resistant to 5-FC (JUND, et al.. Journal of Bacteriology. 1970, no.102, p.607-15. ; KILLSTRUP, et al.. Journal of Bacteriology. 1989, no.171, p.2124-2127.). By contrast, mammalian cells into which the sequences encoding CDase activity were transferred became sensitive to 5-FC (HUBER, et al.. Cancer Research. 1993, no.53, p.4619-4626. ; MULLEN, et al.. Proceedings of the National Academy of Sciences of the United States of America. 1992, no.89, p.33-37. ; WO 93/01281 (US HEALTH)). In addition, the neighboring, untransformed cells also become sensitive to 5-FC (HUBER, et al.. Proceedings of the National Academy of Sciences of the United States of America. 1994, no.91, p.8302-6.). This phenomenon, which is termed a bystander effect, is due to the cells which are expressing the CDase activity secreting 5-FU, which then intoxicates the neighboring cells by straightforward diffusion across the plasma membrane. This property of 5-FU in diffusing passively represents an advantage as compared with the tk/GCV reference system, where the bystander effect requires there to be contact with the cells which are expressing tk (MESNIL, et al.. Proceedings of the National Academy of Sciences of the United States of America. 1996, no.93, p.1831-35.). All the advantages which CDase offers within the context of gene therapy, in particular anticancer gene therapy, can therefore be readily understood.

The *Saccharomyces cerevisiae* (*S. cerevisiae*) FCY1, Candida Albicans FCA1 and the E. coli codA genes, which respectively encode the CDase of these two organisms, are known and their sequences have been published (SEQ ID N°:4; SEQ ID N°:5; SEQ ID N°:6 respectively).

With this respect, according to a more preferred embodiment of the invention, the gene coding a protein having a CDase activity is FCY1, FCA1 or CodA or an analogue thereof. Analogues of these genes refers to a gene having an nucleic acid sequence which have at least a degree of identity greater than 70%, advantageously greater than 80%, preferably greater than 90%, and most preferably greater than 95% with the nucleic acid sequence of the parent gene.

Patent WO 2005/007857 discloses a gene coding a protein having an improved CDase activity. This polypeptides derived from a native CDase by addition of an amino acid sequence. According to another preferred embodiment of the invention, the protein having a CDase activity is a polypeptide disclosed WO 2005/007857 and more preferably the FCU1-8 polypeptide represented in the sequence identifier SEQ ID N°:2 and analogues thereof.

In prokaryotes and lower eukaryotes, uracil is transformed into UMP by the action of uracil phosphoribosyl transferase (UPRTase). This enzyme converts 5-FU into 5-FUMP. According to another preferred embodiment of the invention, the suicide gene codes a protein having an UPRTase activity.

The UPRTase in question may be of any origin, in particular of prokaryotic, fungal or yeast origin. By way of illustration, the nucleic acid sequences encoding the UPRTases from *E*. *coli*(ANDERSEN, et al. Characterization of the upp gene encoding uracil phosphoribosyltransferase of Escherichia coli K12. European Journal ofBiochemistry. 1992, no.204, p.51-56.), from *Lactococcus lactis* (MARTINUSSEN, et al. Cloning and characterization of upp, a gene encoding uracil phosphoribosyltransferase from Lactococcus lactis. Journal of Bacteriology. 1994, vol.176, no.21, p.6457-63.), from *Mycobacterium bovis* (KIM, et al. Complete sequence of the UPP gene encoding uracil phosphoribosyltransferase from Mycobacterium bovis BCG. Biochemistry and molecular biology international. 1997, vol.41, no.6, p.1117-24.) and from *Bacillus subtilis* (MARTINUSSEN, et al. Two genes encoding uracil phosphoribosyltransferase are present in Bacillus subtilis. Journal of Bacteriology. 1995, vol.177, no.1, p.271-4.) may be used in the context of the invention. However, it is most particularly preferred to use a yeast UPRTase and in particular that encoded by the *S. cerevisiae FUR1* gene whose sequence disclosed in KERN, et al. The FUR1 gene of Saccharomyces cerevisiae: cloning, structure and expression of wild-type and mutant alleles. (Gene. 1990, vol.88, no.2, p.149-57.) is introduced here by way of reference. As a guide, the sequences of the genes and those of the corresponding UPRTases may be found in the literature and the specialist databanks (SWISSPROT, EMBL, Genbank, Medline and the like).

Application EP 0998568 A describes an *FUR1* gene lacking 105 nucleotides in 5' of the coding part allowing the synthesis of a UPRTase from which the 35 first residues have been deleted at the N-terminal position and starting with the methionine at position 36 in the native protein. The product of expression of the mutant gene, designated *FUR1Δ105,* is capable of complementing an *fur1* mutant of *S. cerevisiae.* In addition, the truncated mutant exhibits a higher UPRTase activity than that of the native enzyme. Thus, according to a particularly advantageous embodiment of the invention, the suicide gene codes a deletion mutant of a native UPRTase. The deletion is preferably located in the N-terminal region of the original UPRTase. It may be complete (affecting all the residues of said N-terminal region) or partial (affecting one or more continuous or discontinuous residues in the primary structure). In general, a polypeptide consists of N-terminal, central and C-terminal parts, each representing about a third of the molecule. For example, since the *S. cerevisiae* UPRTase has 251 amino acids, its N-terminal part consists of the first 83 residues starting with the so-called initiator methionine situated at the first position of the native form. As for the *E*. *coli* UPRTase, its N-terminal part covers positions 1 to 69.

A preferred protein having an UPRTase activity comprises an amino acid sequence substantially as represented in the sequence identifier SEQ ID N°: 1 of EP 0998568 A , starting with the Met residue at position 1 and ending with the Val residue at position 216. The term "substantially" refers to a degree of identity with said sequence SEQ ID N°: 1 EP 0998568 A greater than 70%, advantageously greater than 80%, preferably greater than 90%, and most preferably greater than 95%. More preferably still, it comprises the amino acid sequence represented in the sequence identifier SEQ ID N°: 1 EP 0998568 A. As mentioned above, it may comprise additional mutations. There may be mentioned in particular the substitution of the serine residue at position 2 (position 37 in the native UPRTase) by an alanine residue.

According to another preferred embodiment of the invention, the suicide gene codes a protein having at least one CDase and one UPRTase activity. Patent applications WO 96/16183 and EP 0998568 A describe the use of a fusion protein encoding an enzyme with two domains having the CDase and UPRTase activities and demonstrate that the transfer of a hybrid gene *codA::upp* or *FCY1::FUR1* or *FCY1::FUR1Δ105 (i.e. FCU1)* carried by an expression plasmid increases the sensitivity of the transfected B16 cells to 5-FC. According to a more preferred embodiment of the invention, the suicide gene codes a polypeptide comprising an amino acid sequence substantially as represented in the sequence identifier SEQ ID N°:3 (coda::upp), SEQ ID N°:1 (FCU1) or FCY1::FUR1. The term "substantially" refers to a degree of identity with said sequence greater than 70%, advantageously greater than 80%, preferably greater than 90%, and most preferably greater than 95%. More preferably still, it comprises the amino acid sequence as represented in the sequence identifier SEQ ID N°:3 (coda::upp), SEQ ID N°:1 (FCU1) or FCY1::FUR1. As mentioned above, it may comprise additional mutations.

The nucleic acid sequences may be easily obtained by cloning, by PCR or by chemical synthesis according to the conventional techniques in use. They may be native genes or genes derived from the latter by mutation, deletion, substitution and/or addition of one or more nucleotides. Moreover, their sequences are widely described in the literature which can be consulted by persons skilled in the art.

Persons skilled in the art are capable of cloning the CDase or UPRTase sequences from the published data and of carrying out possible mutations, of testing the enzymatic activity of the mutant forms in an acellular or cellular system according to the prior art technology or based on the protocol indicated in application EP 0998568 A, and of fusing, in particular in phase, the polypeptides with CDase and UPRTase activity, and consequently all or part of the corresponding genes.

According to a more preferred embodiment, the poxvirus for use according to the invention further comprises a nucleic acid sequence comprising a gene coding a permease.

Permease refers to transmembraneous protein involved in the transfer of a drug comprising one nucleobase moiety, or a precursor thereof through the cell membrane.

Permease comprises but are limited to purine permease, cytosine permease and nucleoside transporters.

According to a preferred embodiment of the invention, permease is a purine or a cytosine permease of *S. Cerevisiae.* The nucleobase transporters of *S. cerevisiae* consist of the purine-cytosine permease, known as FCY2, and the uracil permease, known as FUR4 . The purine-cytosine permease, FCY2 mediates symport of protons and adenine, guanine, hypoxanthine and cytosine across the yeast plasma membrane (Grenson 1969, Jund and Lacroute 1970, Polak and Grenson 1973, Chevallier et al. 1975, Hopkins et al. 1988). FCY2 protein mediates also the transport of 5-fluorocytosine, an analogue of cytosine (Grenson 1969, Jund and Lacroute 1970). FCY2 gene encodes a protein of 533 amino acids (58 kDa) initially predicted to have 10-12 transmembrane-spanning domains (Weber et al. 1990), with nine now favoured (Ferreira et al. 1999). FCY2 exhibits similar affinities for the purine nucleobases and cytosine (Brethes et al. 1992). Uracil uptake into *S. cerevisiae* is mediated by the uracil permease, FUR4 (Jund and Lacroute 1970, Jund et al. 1977). FUR4 is a uracil-proton symporter (Hopkins et al. 1988) predicted to be a protein of 633 amino acids (71.7 kDa) with 10 transmembrane domains and long cytoplasmic hydrophylic N- and C-terminal tails (Jund et al. 1988, Garnier et al. 1996). FUR4 protein can also mediates the transport of 5-fluorouracil, an analogue of uracil (Jund and Lacroute 1970).

Amino acid sequences of FCY2 and Fur4 are notably available in the swissprot database (accession number P17064 and P05316 respectively). Preferably, permease has an amino acid sequence chosen from the group comprising amino acid sequence SEQ ID N0:1 and SEQ ID N0:2 as disclosed in patent application WO 2006/048768.

With this respect, according to a preferred embodiment of the invention, the permease is chosen from the group comprising FCY2 and Fur4 and analogues thereof. Analogues of Fur4 and FCY2 refers to polypeptide having an amino acid sequence which have at least a degree of identity greater than 70%, advantageously greater than 80%, preferably greater than 90%, and most preferably greater than 95% with the amino acid sequence of the parent protein as described here above and which retains the ability to transport a drug comprising one nucleobase moiety through the cell membrane.

The one skilled in the art is able to choose the permease which will be associated with the drug or the precursor of the drug comprising one nucleobase moiety. For example, FCY2 and Fur4 are preferably associated with 5-Fluorocytosine (5-FC).

According to a more preferred embodiment, the poxvirus for use according to the invention may further comprise the elements necessary for the expression of the nucleic acid of interest.

According to a more preferred embodiment, the poxvirus for use according to the invention may further comprise the elements necessary for the expression of the nucleic acid sequence comprising a gene coding a permease.

These elements necessary for the expression of the nucleic acid of interest and/or the nucleic acid sequence comprising a gene coding a permease comprised the elements required for transcription of said DNA into mRNA and, if necessary, for translation of mRNA into polypeptide. Transcriptional promoters suitable for use in various vertebrate systems are widely described in literature. For example, suitable promoters include viral promoters like RSV, MPSV, SV40, CMV or 7.5k, vaccinia promoter, inducible promoters, etc. Preferred promoters are isolated from poxviruses e.g. 7.5K, H5R, TK, p28, p11 or K1L of vaccinia virus. Alternatively, one may use a synthetic promoter such as those described in CHAKRABARTI. . Biotechniques. 1997, no.23, p.1094-97. , HAMMOND, et al.. Journal of Virological Methods. 1997, no.66, p.135-38. and KUMAR.. Virology. 1990, no.179, p.151-8. as well as chimeric promoters between early and late poxviral promoters.

The nucleic acid sequence of interest and the nucleic acid sequence comprising a gene coding a permease may further include additional functional elements, such as intron sequences, targeting sequences, transport sequences, secretion signal, nuclear localization signal, IRES, poly A transcription termination sequences, tripartite leader sequences, sequences involved in replication or integration. Said sequences have been reported in the literature and can be readily obtained by those skilled in the art.

Also disclosed herein is a process for preparing a poxvirus for use according to the invention, in which process:
(i) a poxvirus for use according to the invention is introduced into a cell,
(ii) said cell is cultured under conditions which are appropriate for enabling said poxvirus to be produced, and
(iii) said poxvirus is recovered from the cell culture.

While the poxvirus can of course be recovered from the culture supernatant, it can also be recovered from the cells. One of the commonly employed methods consists in lysing the cells by means of consecutive freezing/thawing cycles in order to collect the virions in the lysis supernatant. The virions can then be amplified and purified using the techniques of the art (chromatographic method, method of ultra-centrifugation, in particular through a cesium chloride gradient, etc.).

The composition for use according to the invention may comprise a pharmaceutically acceptable excipient.

A composition for use according to the invention is more specifically intended for the preventive or curative treatment of diseases by means of gene therapy and is more specifically aimed at proliferative diseases (cancers, tumors, restenosis, etc.) or aimed at diseases associated to an increased osteoclast activity (e.g. rheumatoid arthritis, osteoporosis).

A composition for use according to the invention can be made conventionally with a view to administering it locally, parenterally or by the digestive route. In particular, a therapeutically effective quantity of the recombinant vector or poxvirus for use according to the invention is combined with a pharmaceutically acceptable excipient. It is possible to envisage a large number of routes of administration. Examples which may be mentioned are the intragastric, subcutaneous, intracardiac, intramuscular, intravenous, intraperitoneal, intratumor, intranasal, intrapulmonary and intratracheal routes. In the case of these three latter embodiments, it is advantageous for administration to take place by means of an aerosol or by means of instillation. The administration can take place as a single dose or as a dose which is repeated on one or more occasions after a particular time interval. The appropriate route of administration and dosage vary depending on a variety of parameters, for example the individual, the disease to be treated or the gene(s) of interest to be transferred. The preparations based on viral particles for use according to the invention can be formulated in the form of doses of between 10⁴ and 10¹⁴ pfu (plaque-forming units), advantageously 10⁵ and 10¹³ pfu, preferably 10⁶ and 10¹² pfu, more preferably 10⁶ and 10⁷.

The composition can also include a diluent, an adjuvant or an excipient which is acceptable from the pharmaceutical point of view, as well as solubilizing, stabilizing and preserving agents. In the case of an injectable administration, preference is given to a formulation in an aqueous, nonaqueous or isotonic solution. It can be presented as a single dose or as a multidose, in liquid or dry (powder, lyophilizate, etc.) form which can be reconstituted at the time of use using an appropriate diluent.

Also disclosed is the use of a poxvirus or a composition as described herein for preparing a medicament which
is intended for treating the human or animal body by gene therapy. The medicament can be administered directly *in vivo* (for example by intravenous injection, into an accessible tumor, into the lungs by means of an aerosol, into the vascular system using an appropriate catheter, etc.).

A preferred use consists in treating or preventing cancers, tumors and diseases which result from unwanted cell proliferation. Conceivable applications which may be mentioned are cancers of the breast, of the uterus (in particular those induced by papilloma viruses), of the prostate, of the lung, of the bladder, of the liver, of the colon, of the pancreas, of the stomach, of the oesophagus, of the larynx, of the central nervous system (e.g. glioblastoma) and of the blood (lymphomas, leukemia, etc.). An other preferred use consists in treating or preventing rheumatoid arthritis, osteoporosis and other diseases associated to an increased osteoclast activity. It can also be used in the context of cardiovascular diseases, for example in order to inhibit or retard the proliferation of the smooth muscle cells of the blood vessel wall (restenosis). Finally, in the case of infectious diseases, it is possible to conceive of the medicament being applied to AIDS.

When the composition for use according to the invention is used for the treatment of cancer, the preferred route of administration is the systemic route since the poxvirus is able to specifically target the tumoral cells.

Also disclosed is a method for treating diseases characterized in that a poxvirus, a composition as described is administered to an host organism or cell which is in need of such treatment.

According to an advantageous embodiment, the therapeutic use or the treatment method also comprises an additional step in which pharmaceutically acceptable quantities of a prodrug, advantageously an analog of cytosine, in particular 5-FC, are administered to the host organism or cell. By way of illustration, it is possible to use a dose of from 50 to 500 mg/kg/day, with a dose of 200 mg/kg/day or of 100 mg/kg/day being preferred. Within the context of the present invention, the prodrug is administered in accordance with standard practice (e.g. per os, systematically).

Preferably, the administration taking place subsequent to the administration of the therapeutic agent for use according to the invention, preferably at least 3 days, more preferably at least 4 days and even more preferably at least 5 days after the administration of the therapeutic agent. According to an even more preferred embodiment of the invention, the administration of the prodrug takes place 7 days after the administration of the therapeutic agent. The oral route is preferred. It is possible to administer a single dose of prodrug or doses which are repeated for a time which is sufficiently long to enable the toxic metabolite to be produced within the host organism or cell.

Furthermore, the composition for use according to the invention can be combined with one or more substances which potentiate the cytotoxic effect of the 5-FU. Mention may in particular be made of drugs which inhibit the enzymes of the pathway for the *de novo* biosynthesis of the pyrimidines (for example those mentioned below), drugs such as Leucovorin (Waxman et al., 1982, Eur. J. Cancer Clin. Oncol. 18, 685-692), which, in the presence of the product of the metabolism of 5-FU (5-FdUMP), increases the inhibition of thymidylate synthase, resulting in a decrease in the pool of dTMP, which is required for replication, and finally drugs such as methotrexate (Cadman et al., 1979, Science 250, 1135-1137) which, by inhibiting dihydrofolate reductase and increasing the pool of PRPP (phosphoribosylpyrophosphate), brings about an increase in the incorporation of 5-FU into the cellular RNA.

In the context of the present invention, the drugs which inhibit the enzymes of the pathway for the *de novo* biosynthesis of the pyrimidines are preferably selected from the group consisting of PALA (N-(phosphonoacetyl)-L-aspartate; Moore etal., 1982, Biochem. Pharmacol. 31, 3317-3321), Leflunomide, A771726 (active metabolite of Leflunomide; Davis et al., 1996, Biochem. 35, 1270-1273) and Brequinar (Chen et al., 1992, Cancer Res. 52, 3251-3257).

The composition or method as described can be combined with one or more substances effective in anticancer therapy. Among pharmaceutical substances effective in anticancer therapy which may be used in association or in combination with the compositions for use according to the invention, there may be mentioned alkylating agents such as, e.g., mitomycin C, cyclophosphamide, busulfan, ifosfamide, isosfamide, melphalan, hexamethylmelamine, thiotepa, chlorambucil, or dacarbazine; antimetabolites such as, e.g., gemcitabine, capecitabine, 5-fluorouracil, cytarabine, 2-fluorodeoxy cytidine, methotrexate, idatrexate, tomudex or trimetrexate; topoisomerase II inhibitors such as, e.g., doxorubicin, epirubicin, etoposide, teniposide or mitoxantrone; topoisomerase I inhibitors such as, e.g., irinotecan (CPT-11), 7-ethyl-10-hydroxy-camptothecin (SN-38) or topotecan; antimitotic drugs such as, e.g., paclitaxel, docetaxel, vinblastine, vincristine or vinorelbine; and platinum derivatives such as, e.g., cisplatin, oxaliplatin, spiroplatinum or carboplatinum.

The one or more substances effective in anticancer therapy and the composition for use according to the invention may be administered once or several times by methods well known to the skilled practitioner including systemic administration and direct injection into the tumor. One may proceed by bolus injection or perfusion (e.g. over 0.5-6 hours). The various administrations of the anti-cancer substance and/or the poxvirus composition can be separated each other by an appropriate period of time and carried out by the same route or by different routes of administrations at the same site or at different sites using the same or different dosages. The dosage may be adapted by the practitioner in light of various parameters such as the nature of the cancer to be treated, the mode and the frequency of administration(s), and other factors routinely considered in administering a drug.

In one embodiment, the poxvirus for use according to the invention is combined with the topoisomerase I inhibitor irinotecan. Preferably, irinotecan and the poxvirus composition will be administered intravenously. By way of illustration, suitable doses for irinotecan may vary from 5 to 100 mg/kg/day and those for poxvirus from 1x106 to 1x109 pfu. Preferably, poxvirus composition will be injected once to three times intravenously and irinotecan at a suitable periodicity (e.g. once a week) for one or more cycles. Additionally, irinotecan treatment will be carried out a few days (e.g. 3 to 10 days) after the first administration of the poxvirus composition.

In another embodiment, the poxvirus for use according to the invention is combined with the antimitotic drug oxaliplatin. Preferably, oxaliplatin will be administered intraperitonally or intravenously and the poxvirus composition for use according to the invention by intravenous route. By way of illustration, suitable doses for oxaliplatin may vary from 0.5 to 10 mg/kg/day and those for poxvirus from 1x106 to 1x109 pfu. Preferably, poxvirus composition will be injected once to three times intravenously and oxaliplatin at a suitable periodicity (e.g. every two weeks) for one or more cycles. Additionally, a preferred method comprises first the administration of the poxvirus composition followed by oxaliplatin treatment few days (e.g. 3 to 10 days) after the first poxvirus injection.

Furthermore, the use according to the invention may also comprise an additional step in which pharmaceutically acceptable quantities of a prodrug are administered to the host organism or cell as described above. A preferred prodrug in the context of the invention is 5-FC.

The compositions for use according to the invention can also be used in combination with radiotherapy.

The compositions for use according to the invention may also be used in combination with one or more other agents including but not limited to immunomodulatory agents such as, e.g. alpha, beta or gamma interferon, interleukin (in particular IL-2, IL-6, IL-10 or IL-12) or tumor necrosis factor; agents that affect the regulation of cell surface receptors such as, e.g. inhibitors of Epidermal Growth Factor Receptor (in particular cetuximab, panitumumab, zalutumumab, nimotuzumab, matuzumab, gefitinib, erlotinib or lapatinib) or inhibitors of Human Epidermal Growth Factor Receptor-2 (in particular trastuzumab); and agents that affect angiogenesis such as, e.g. inhibitor of Vascular Endothelial Growth Factor (in particular bevacizumab or ranibizumab).

### Brief Description of Figures in the Drawings

Figure 1. In vitro Sensitivities to 5-FC of vaccinia viruses infected human colorectal tumor cells (LoVo). LoVo cells, infected at a MOI of 0.0001 with the indicated viruses (mock (●) VVTK-/FCU1 (■) or VVTK-I4L-/FCU1 (Δ)) were exposed to various concentration of 5-FC. Cell survival was measured at 5 days post-infection. Results were expressed in percentage of cellular viability in the presence or not of drugs. Values are represented in mean ± SD of three individual determinations without the cell mortality due to the replication of the viruses.
Figure 2. In vitro Sensitivities to 5-FC of vaccinia viruses infected human colorectal tumor cells (LoVo). LoVo cells, infected at a MOI of 0.0001 with the indicated viruses (mock (●) VVTK-/FCU1 (■) or WTK-F4L-/FCU1 (◊) were exposed to various concentration of 5-FC. Cell survival was measured at 5 days post-infection. Results were expressed in percentage of cellular viability in the presence or not of drugs. Values are represented in mean ± SD of three individual determinations without the cell mortality due to the replication of the viruses.
Figure 3. In vitro replication efficacy of VVTK-/FCU1 and VVTK-I4L-/FCU1 in LoVo infected at a MOI of 0.0001 with the indicated viruses at day 5 post infection. Values are represented in mean ± SD of three individual determinations.
Figure 4. In vitro replication efficacy of VVTK-/FCU1 and VVTK-F4L-/FCU1 in LoVo infected at a MOI of 0.0001 with the indicated viruses at day 5 post infection. Values are represented in mean ± SD of three individual determinations.
Figure 5. Mean tumor volume ± SEM of s.c LoVo in Swiss nude mice after i.v injection of virus. 7 days after inoculation with tumor (palpable tumor), mice were treated by 10⁷ pfu of buffer + saline (◊), buffer + 5-FC (◆), VVTK-I4L-/FCU1 + saline (Δ) or VVTK-I4L-/FCU1 + 5-FC (▲). The animals were treated by saline or 5-FC at 100 mg/kg/j twice a day by oral gavage, 7 days after virus injection during 3 weeks. Tumor volume was measured twice a week.
Figure 6. Mean tumor volume ± SEM of s.c LoVo in Swiss nude mice after i.v injection of virus. 7 days after inoculation with tumor (palpable tumor), mice were treated by 10⁷ pfu of buffer + saline (◊), buffer + 5-FC (◆), VVTK-F4L-/FCU1 + saline (□) or VVTK-F4L-/FCU1 + 5-FC (■). The animals were treated by saline or 5-FC at 100 mg/kg/j twice a day by oral gavage, 7 days after virus injection during 3 weeks. Tumor volume was measured twice a week.

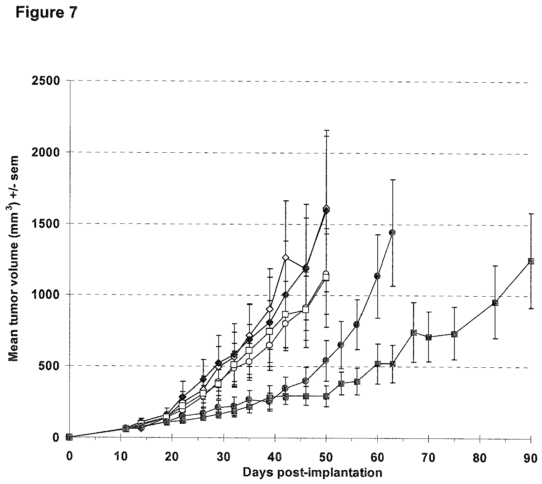

Figure 7. Mean tumor volume ± SEM of s.c LoVo in Swiss nude mice after i.v injection of virus. 11 days after inoculation with tumor (palpable tumor), mice were treated by buffer + H₂O (◆), or buffer + 5-FC (◆), or one injection of 10⁷ pfu of VVTK-I4L-/FCU1 + H₂O (○), or one injection of 10⁷ pfu of VVTK-I4L-/FCU1 + 5-FC (5-FC administrated 7 days after virus injection and during 3 weeks) (●), or two injections (day 11 and day 33) of 10⁷ pfu of VVTK-I4L-/FCU1 + H₂O (□), or two injections (day 11 and day 33) of 10⁷ pfu of VVTK-I4L-/FCU1 + 5-FC (5-FC administrated from day 18 to day 32 and from day 40 to day 54) (■). The animals were treated by 5-FC at 100 mg/kg twice a day by oral gavage. Tumor volume was measured twice a week.
Figure 8. Mean tumor volume ± SEM of s.c U87-MG (glioblastoma tumor cells) in Swiss nude mice after i.v injection of virus. 11 days after inoculation with tumor (palpable tumor), mice were treated by buffer + H₂O (◊), or buffer + 5-FC (◆), or 10⁷ pfu of VVTK-I4L-/FCU1 + H₂O (○), or 10⁷ pfu of WTK-I4L-/FCU1 + 5-FC (●). The animals were treated by 5-FC at 100 mg/kg twice a day by oral gavage, 7 days after virus injection and during 3 weeks. Tumor volume was measured twice a week.
Figure 9. Ratio of virus yield in dividing cells versus in confluent cells. PANC1 (pancreatic human tumor), H1299 (Lungs human tumor) or U118MG (glioma human tumor) cells are infected with 100 pfu of ( 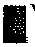 ) VVTK-/FCU1 or ( 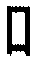 ) VVTK-I4L-/FCU1. 48h post-infection, viral titers were determined. Values are the ratio between yields of virus in dividing cells versus in confluent cells.
Figure 10. Ratio of virus yield in dividing cells versus in confluent cells. PANC1 (pancreatic human tumor), H1299 (Lungs human tumor) or U118MG (glioma human tumor) cells are infected with 100 pfu of ( 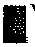 ) VVTK-/FCU1 or ( 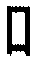 ) VVTK-F4L-/FCU1. 48h post-infection, viral titers were determined. Values are the ratio between yields of virus in dividing cells versus in confluent cells.
Figure 11. Viral titers (pfu/mg of tissue) in organs or tumors at day 6 and day 21 after i.v. infection into Swiss nude mice bearing subcutaneous human tumors with 1x10⁶ PFU of VVTK-/FCU1 ( 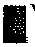 ) or VVTK-I4L-/FCU1 ( 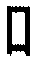 ).
Figure 12. Viral titers (pfu/mg of tissue) in organs or tumors at day 6 and day 21 after i.v. infection into Swiss nude mice bearing subcutaneous human tumors with 1x10⁶ PFU of VVTK-/FCU1 ( 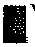 ) or VVTK-F4L-/FCU1 ( 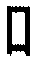 ).
Figure 13. Survival of Swiss nude mice after treatment with 1x10⁸ pfu of VVTK-/FCU1 (■) or VVTK-I4L-/FCU1 (○) by i.v injection.
Figure 14. Survival of immunocompetent B6D2 mice after treatment with 1x10⁷ pfu (A) or 1x10⁸ pfu (B) of VVTK-/FCU1 (■) or VVTK-I4L-/FCU1 (◊) by i.v injection.
Figure 15. Average quantity of pocks on tails after i.v injection of 1x10⁶ pfu VVTK-/FCU1 or VVTK-I4L-/FCU1 in Swiss nude mice at day 13 post-infection and at day 34 post-infection.
Figure 16. Average quantity of pocks on tails after i.v injection of 1x10⁶ pfu VVTK-/FCU1 or VVTK-F4L-/FCU1 in Swiss nude mice at day 13 post-infection and at day 34 post-infection.
Figure 17. Average quantity of pocks on tails after i.v injection of 1x10⁷ pfu VVTK-/FCU1 or vvTK-I4L-/FCU1 in Swiss nude mice at day 15 post-infection and at day 31 post-infection.
Figure 18. Average quantity of pocks on tails after i.v injection of 1x10⁷ pfu VVTK-/FCU1 or VVTK-F4L-/FCU1 in Swiss nude mice at day 15 post-infection and at day 31 post-infection.

### Mode(s) for Carrying Out the Invention

### Examples

### Construction of vector plasmids

A shuttle plasmid for deleting I4L was constructed using the DNA of vaccinia virus strain Copenhagen (accession number M35027) deleted on Thymidine Kinase gene and expressing FCU1 gene under the control of vaccinia synthetic promoter p11K7.5. The DNA flanking regions of I4L were amplified by PCR. Primers of the downstream flanking region of I4L were 5'- TCC CCC GGG TTA ACC ACT GCA TGA TGT ACA -3' (SEQ ID N°:7; *Smalsite* underlined) and 5'- GCC GAG CTC GAG GTA GCC GTT TGT AAT TCT -3' (SEQ ID N°:8; *Sac*/site underlined). Primers for the upstream region were 5'- GCC TGG CCA TAA CTC CAG GCC GTT - 3' (SEQ ID N°:9; *Msc*/site underlined) and 5' - GCC CAG CTG ATC GAG CCG TAA CGA TTT TCA- 3' (SEQ ID N°:10; *PvuII* site underlined). The amplified DNA fragment were digested with restriction enzyme *Smal*/*Sacl* or *MscI*/*PvuII* and ligated into the corresponding sites in *PpolyIIIplasmid.* A repeat region of the downstream flanking region of I4L was amplified by PCR using the primers 5'- GCC GCA TGC ATC CTT GAA CAC CAA TAC CGA - 3' (SEQ ID N°:11; *SphI*site underlined) and 5'- GCT CTA GAG AGG TAG CCG TTT GTA ATC TG - 3' (SEQ ID N°:12; *XbaIsite* underlined) and inserted in *PpolyIII* plasmid*.* The repeat region is used to eliminate the selection cassette during the production of deleted viruses. The selection cassette, corresponding to the GFP/GPT fusion gene under the control of pH5R vaccinia promoter, was inserted into the *SacI*/*SphI* site in *PpolyIII* plasmid. The obtained plasmid is the recombinant shuttle plasmid named pΔI4L for deletion of I4L gene.

A shuttle plasmid for deleting F4L was constructed using the DNA of vaccinia virus strain Copenhagen (accession number M35027). The DNA flanking regions of F4L were amplified by PCR. Primers of the downstream flanking region of F4L were 5'- CGC GGA TCC TTT GGT ACA GTC TAG TAT CCA - 3' (SEQ ID N°:13; *BamHI* site underlined) and 5' - TCC CCC GGG TTA TAA CAG ATG CAG TAT CCA - 3' (SEQ ID N°:14; *Smalsite* underlined). Primers for the upstream region were 5'- GCC CAG CTG TTC AAT GGC CAT CTG AAA TCC - 3' (SEQ ID N°:15; *PvuII* site underlined) and 5'- GAA GAT CTA GTA TCG CAT CTA AAA GAT GG - 3' (SEQ ID N°:16; *BgIII* site underlined). The amplified DNA fragment were digested with restriction enzyme *BamHI*/*Smal* or *BgIII*/*PvuII* and ligated into the corresponding sites in *PpolyIII* plasmid. A repeat region of the downstream flanking region of I4L was amplified by PCR using the primers 5'- GCC GAG CTC ACC CAC ACG TTT TTC GAA AAA - 3' (SEQ ID N°:17; *SacI* site underlined) and 5'- GCC GCA TGC TTA TAA CAG ATG CAG TAT CAA - 3' (SEQ ID N°:18; *SphI* site underlined) and inserted in *PpolyIII* plasmid. The repeat region is used to eliminate the selection cassette during the production of deleted viruses. The selection cassette, corresponding to the GFP/GPT fusion gene under the control of pH5R vaccinia promoter, was inserted into the *SacI*/*SmaI* site in *PpolyIII* plasmid. The obtained plasmid is the recombinant shuttle plasmid named pΔF4L for deletion of F4L gene.

### The generation of recombinant vaccinia viruses.

CEF cells were infected with VVTK-FCU1 (Vaccinia virus, defective for the J2R *Kinase* gene, expressing *FCU1* gene under the control of synthetic promoter p11k7.5) strain Copenhagen at a MOI of 0.1 and incubated at 37°C for 2 h, then transfected with a CaCl₂ coprecipitate of the recombinant shuttle plasmid (0.2 µg). The cells were incubated for 48 h at 37°C. Dilutions of virus emerging were then used to infect the CEF cells in selection medium containing Hypoxanthine at final concentration of 15 µg/ml, xanthine at final concentration of 250 µg/ml and mycophenolic acide at final concentration of 250 µg/ml. Fluorescent (GFP) and positive (GPT selection) plaques were isolated and selected for a several round of selection in CEF cells in presence of GPT selection medium. The presence or not of VVTK-FCU1 was determined by 40 cycles of PCR with primers inside the deletion region. After the elimination of parental virus, the double deleted virus was used to infect CEF without GPT selection medium to eliminate the selection cassette. Non-fluorescent plaques were isolated and selected for 2 cycles in CEF. Final recombinant VV viruses were amplified in CEF, purified and virus stocks were titrated on CEF by plaque assay.

### In vitro cell sensitivity to 5-FC.

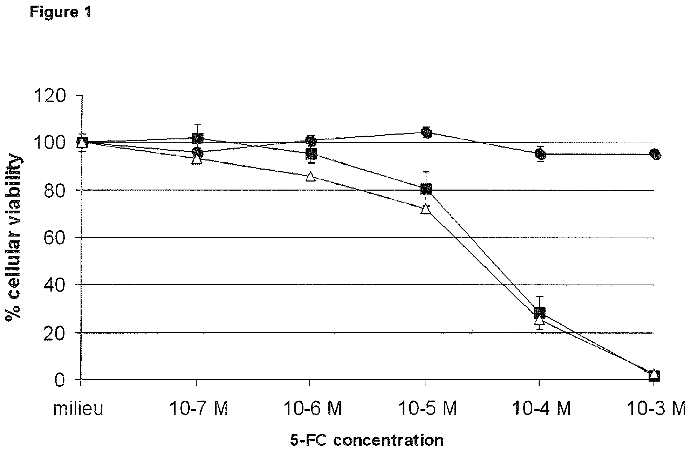

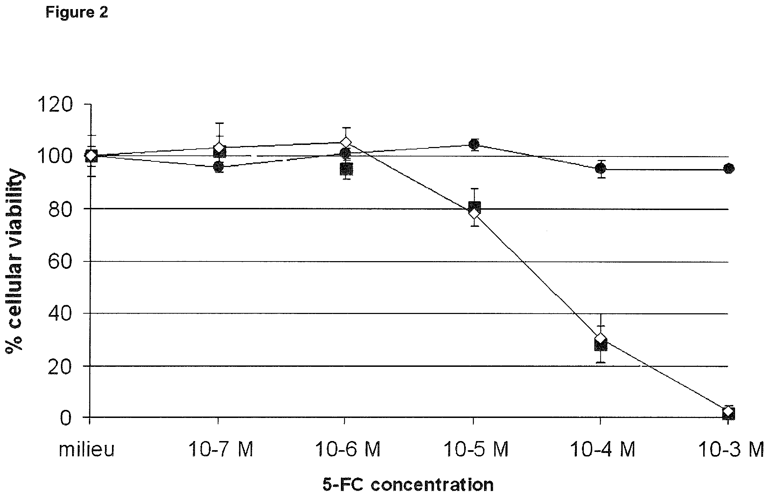

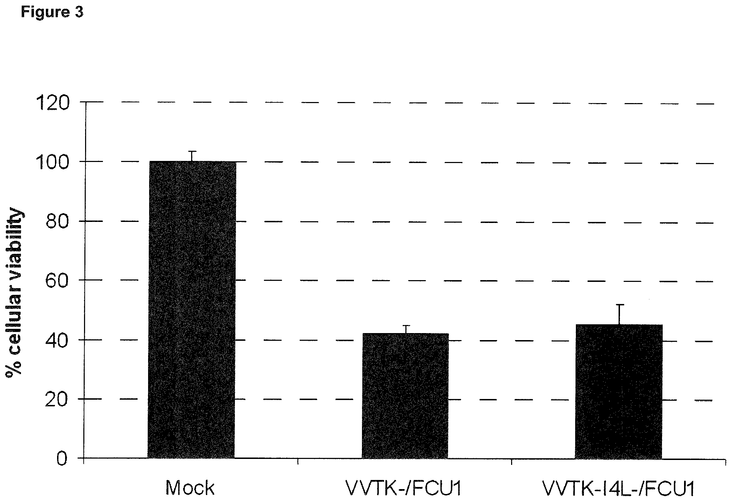

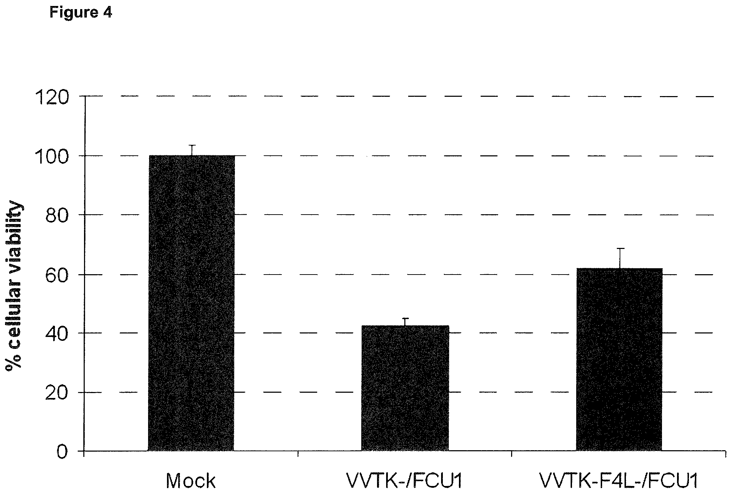

Human tumor cells were transduced by the respective recombinant VV at a MOI of 0.0001. A total of 3 x 10⁵ cells/well were plated in 6-well culture dishes in 2 ml of medium containing various concentrations of 5-FC. Cells were then cultured at 37°C for 5 days, and the viable cells were counted by trypan blue exclusion. Results depicted in figure 1, 2, 3 and 4 shows that the FCU1 activity is equivalent in viruses defective for the J2R gene than in viruses defective for the I4L and J2R gene or than in viruses defective for the F4L and J2R gene.

### In vitro replication in cultured cells.

Dividing or confluent cells were infected, in 6-wells plaques, at 100 PFU of viruses (nearly MOI 0.0005). 2 mL of medium supplemented with 10% FCS for dividing cells and no supplemented for confluent cells were added. The cells were harvested at 48 hours post-infection. The cells were stored at -20°C and sonicated to release the virus, virus was also quantified by plaque titration on CEF cells. The ratio between replication in dividing cells and confluent cells are similar in all cells. Both viruses VVTK-/FCU1, VVTK-I4L-/FCU1 and VVTK-F4L-/FCU1 replicate more in dividing cells than in confluent cells.

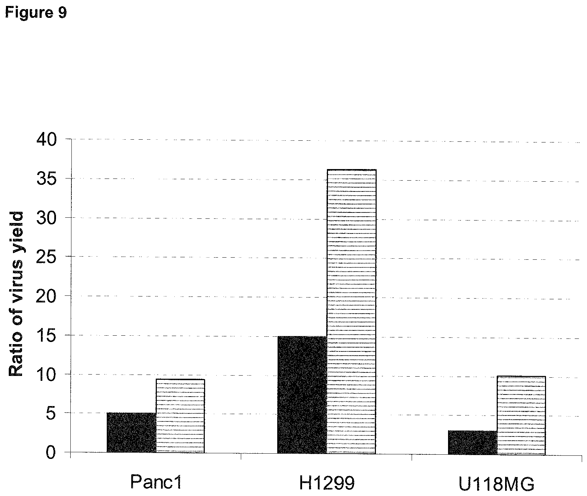

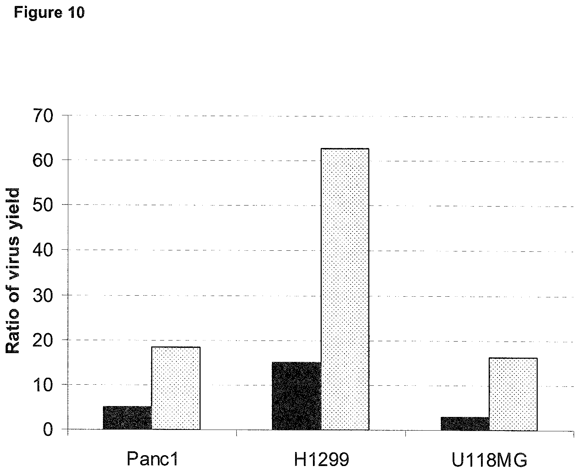

As an indirect mean to assay for replication virus specificity, the yield of virus produced in dividing versus confluent tumor cells (pancreatic human tumor PANC1; lung human tumor H1299; glioma human tumor U118MG) was determined. Confluent cells were plated at 1x10⁶ cells/well and cultured in complete media for 7 days then 1 day before infection the cells were washed and cultured in media without serum. Dividing cells were plated at 3x10⁵ cells/well one day before infection. To evaluate the level of cell division, the amount of titrated thymidine incorporated into nucleic acid was measured 5 hours, 24 hours and 48 hours after plating cells. During this period thymidine incorporation was relatively constant in confluent cells whereas in dividing cells an increase in incorporation was seen over time. Then the cells were infected with 100 pfu of viruses, and 48h post infection the ratio between the yield of virus produced in dividing tumor cells and in confluent tumor cells was determined by plaque titration on CEF. Results depicted in figures 9 and 10 show that both viruses VVTK-/FCU1, VVTK-I4L-/FCU1 and VVTK-F4L-/FCU1 replicate more in dividing cells than in confluent cells. Results depicted in figures 9 and 10 show moreover an increase of ratio in all the different types of cells for both viruses VVTK-I4L-/FCU1 and VVTK-F4L-/FCU1 by comparison with VVTK-/FCU1. This increase of ratio in all the different types of cells is due to a lower replication of both viruses VVTK-I4L-/FCU1 and VVTK-F4L-/FCU1 in confluent cells. These results demonstrate that both viruses VVTK-I4L-/FCU1 and VVTK-F4L-/FCU1 display an increased specificity toward dividing cells compared to VVTK-/FCU1.

### Subcutaneous tumor model.

Female Swiss nude mice were obtained from Charles River Laboratories. Animals used in the studies were uniform in age (6 weeks) and body weights ranged from 23-26 g. Swiss nude mice were injected subcutaneously (s.c.) into the flank with 5x10⁶ LoVo cells. When tumors reached a diameter of 50-70 mm³, the mice were randomized in a blinded manner and treated with the indicated vectors for the *in vivo* experiments. Biodistribution of the virus.

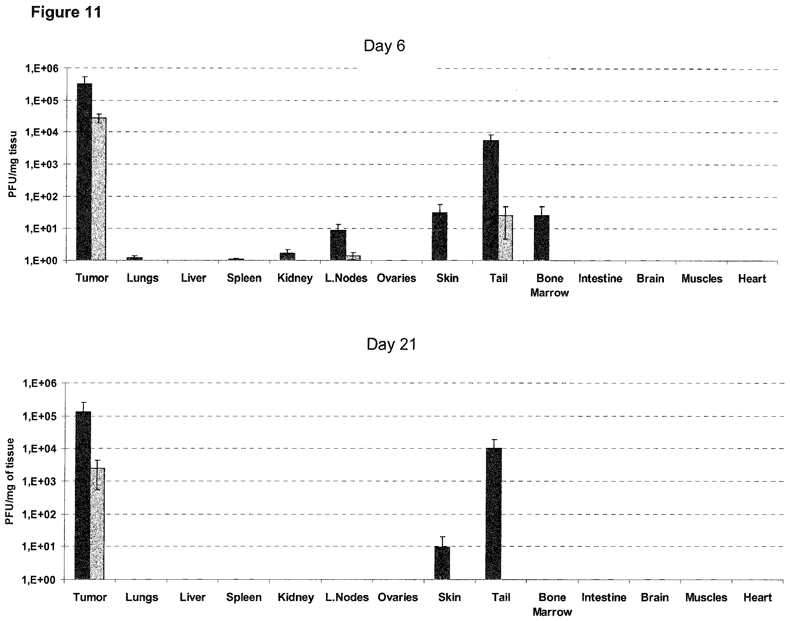

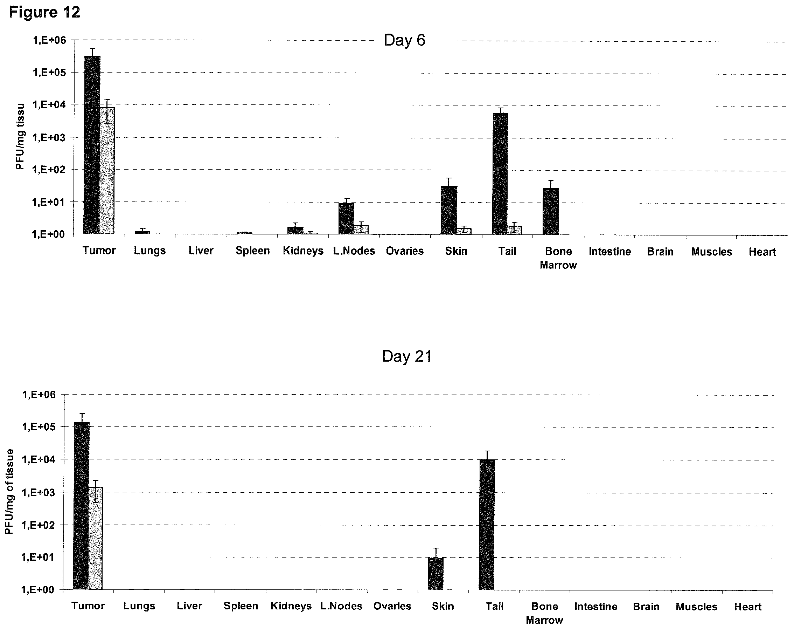

The presence of the various viruses was evaluated by virus titration in tumors and organ samples. 1x10⁶ PFU of VV-FCU1 or VVTK-I4L-/FCU1 or VVTK-F4L-/FCU1 was injected intravenously (i.v.) by tail vein injection into nude mice bearing established s.c. LoVo tumors. Mice were sacrificed at indicated time points, and the tumors and other organs were collected and weighted. Tumors and organs were homogenized in PBS and titers were determined on CEF as described previously. Viral titers were standardized to milligram of tissue. Viral titers were standardized to milligram of tissue. Results depicted in Table 2, 3, 4 and 5 (the range of virus titers is presented in pfu/mg of tissue) show that after 14 days the virus according to the invention is mostly found in the tumor. Results depicted in figures 11 and 12 show that both viruses VVTK-/FCU1, VVTK-I4L-/FCU1 and VVTK-F4L-/FCU1 target the tumor with about 1 000 to 10 000 fold more virus in the tumor than in the other organs analyzed except for tails in the case of VVTK-/FCU1. A small amount of VVTK-/FCU1 is detected in lungs, spleen, kidney and lymph nodes (less than 10 pfu/mg) and more in skin, tail and bone marrow at day 6, and skin and tail at day 21. In contrast, both VVTK-I4L-/FCU1 and VVTK-F4L-/FCU1 have higher tumor specificity with only a small amount in lymph nodes and tail at day 6, and only in tumor at day 21.

**Table 2**

| | Tumor | Lungs | Spleen | Kidney | L.Nodes | Heart |
|---|---|---|---|---|---|---|
| VVTK-/FCU1 | (0,2-3,3)x10⁵ | 0.1-2 | 0-2.2 | 0-1.8 | 0-61 | 0-0.3 |
| VVTK-I4L/FCU1 | 25.6-2.2x10⁵ | 0-0.1 | n.d | 0-1 | n.d | n.d |

**Table 3**

| | Ovaries | Skin | Tail | Bone Marrow | Brain | Muscles |
|---|---|---|---|---|---|---|
| VVTK-/FCU1 | 2.2-74 | 0.1-24 | 13.5-7.10⁴ | 0-800 | 0-1.8 | 0-22 |
| VVTK-I4L/FCU1 | 0-102 | n.d | 26,3 | n.d | n.d | n.d |

**Table 4**

| | Tumor | Lungs | Spleen | Kidney | L.Nodes | Ovaries |
|---|---|---|---|---|---|---|
| VVTK-/FCU1 | (0,2-3,3)x10⁵ | 0.1-2 | 0-2.2 | 0-1.8 | 0-61 | 2.2-74 |
| VVTK-F4L/FCU1 | 51.8-3.8x10⁴ | n.d | n.d | n.d | 0-2.1 | n.d |

**Table 5**

| | Tail | Bone Marrow | Intestine | Brain | Muscles | Heart |
|---|---|---|---|---|---|---|
| VVTK-/FCU1 | 13.5-7.10⁴ | 0-800 | n.d | 0-1.8 | 0-22 | 0-0.3 |
| VVTK-F4L/FCU1 | 0-7.9 | n.d | n.d | n.d | n.d | n.d |

### Antitumor activity of of the poxvirus of the invention in s.c. tumor model.

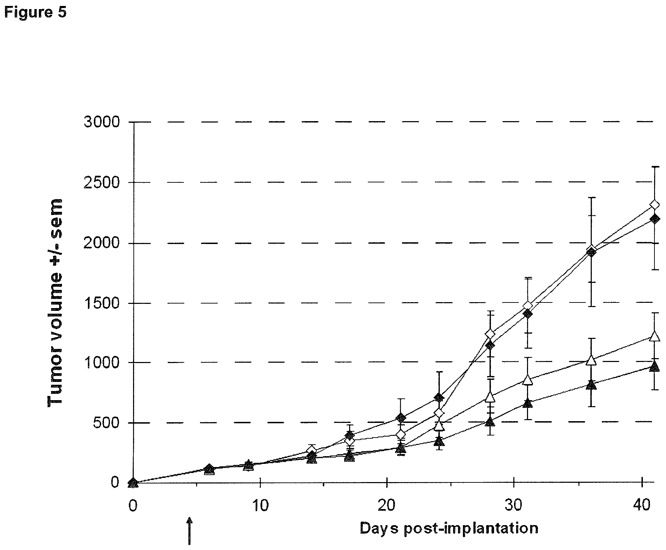

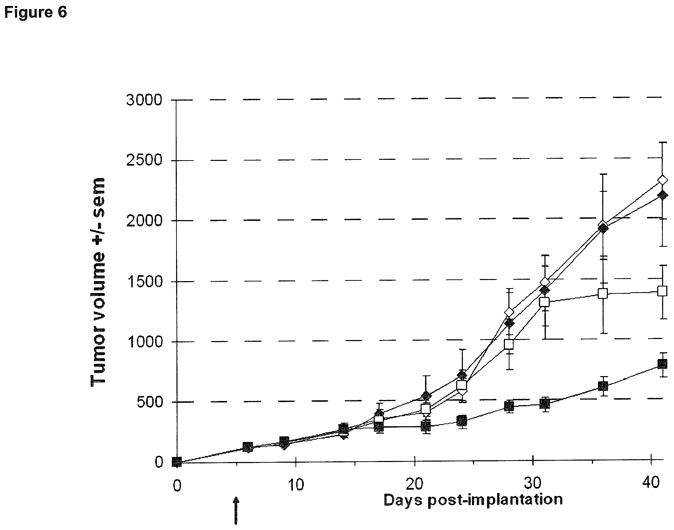

Nude mice bearing established s.c. LoVo tumors (50-70 mm³) were treated one time intravenously (by tail vein) with the indicated vectors at dose of 1.10⁷ PFU, respectively. Starting day 7 following viral injection, 5-FC was given by oral gavage at 100 mg/kg (0.5 ml 5-FC 0.5% in water) twice a day for 3 weeks. Tumor size was measured twice weekly using calipers. Tumor volume were calculated in mm³ using the formula (*p*/6) (length x width²). The results depicted in figure 5 and 6 show that the variouses viruses have a similar efficacy with an oncolytic activity (p<0.05) able to control the growth of tumor, and a combined activity (oncolytic of the virus and therapeutic of FCU1 gene) with administration of 5-FC which can further improve the control of the tumor growth (p<0.01).

Nude mice bearing established s.c. LoVo tumors (50-70 mm³) were also treated intravenously (by tail vein) with the indicated vectors at dose of 1.10⁷ PFU according to the followings: 11 days after inoculation with tumor (palpable tumor), mice were treated by buffer + H₂O, or buffer + 5-FC, or one injection of 10⁷ pfu of VVTK-I4L-/FCU1 + H₂O, or one injection of 10⁷ pfu of VVTK-I4L-/FCU1 + 5-FC (5-FC administrated 7 days after virus injection and during 3 weeks), or two injections (day 11 and day 33) of 10⁷ pfu of VVTK-I4L-/FCU1 + H₂O, or two injections (day 11 and day 33) of 10⁷ pfu of VVTK-I4L-/FCU1 + 5-FC (5-FC administrated from day 18 to day 32 and from day 40 to day 54). The animals were treated by 5-FC at 100 mg/kg twice a day by oral gavage. Tumor size was measured twice weekly using calipers. Tumor volume were calculated in mm³ using the formula (*p*/6) (length x width²). The results depicted in figure 7 show that no antitumoral activity of virus alone after one or two injections. The addition of 5-FC treatment shows statistically significant inhibition of tumor growth (p<0.05) when compared with vehicle groups and virus alone (without 5-FC) until day 50. As with one single injection, two i.v. injections of VVTK-I4L-/FCU1 + 5-FC demonstrates a significant antitumoral activity when compared with vehicule groups and two injections of virus alone (without 5-FC) (p<0.05). Moreover, a significant difference on tumor evolution is observed from day 56 between one and two injections of virus in combination of 5-FC treatment (p<0.05).

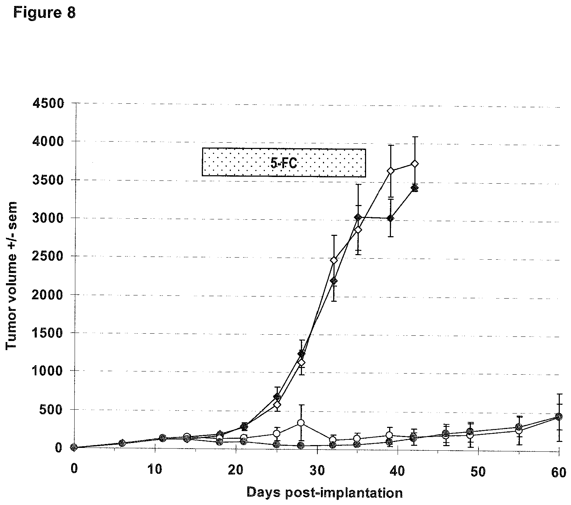

Nude mice bearing established s.c. U87-MG (glioblastoma tumor cells) were treated intravenously (by tail vein) with the indicated vectors at dose of 1.10⁷ PFU according to the followings: 11 days after inoculation with tumor (palpable tumor), mice were treated by buffer + H₂O, or buffer + 5-FC, or 10⁷ pfu of VVTK-I4L-/FCU1 + H₂O, or 10⁷ pfu of VVTK-I4L-/FCU1 + 5-FC. The animals were treated by 5-FC at 100 mg/kg twice a day by oral gavage, 7 days after virus injection and during 3 weeks. Tumor size was measured twice weekly using calipers. Tumor volume were calculated in mm³ using the formula (π/6) (length x width²). The results depicted in figure 8 show a high oncolytic activity of the VVTK-I4L-/FCU1 on U87-MG cells which result in a strong antitumor activity (p<0.0001). The combined activity with addition of 5-FC, by oral gavage, results in similar activity (p<0.0001).

### Viral pathogenicity.

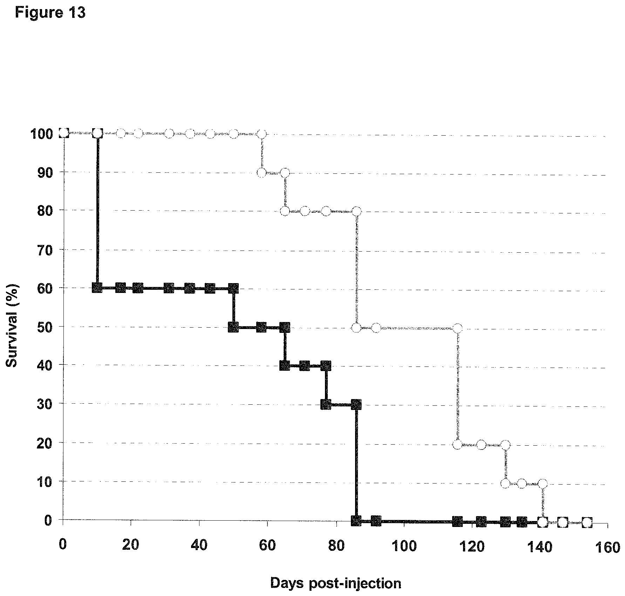

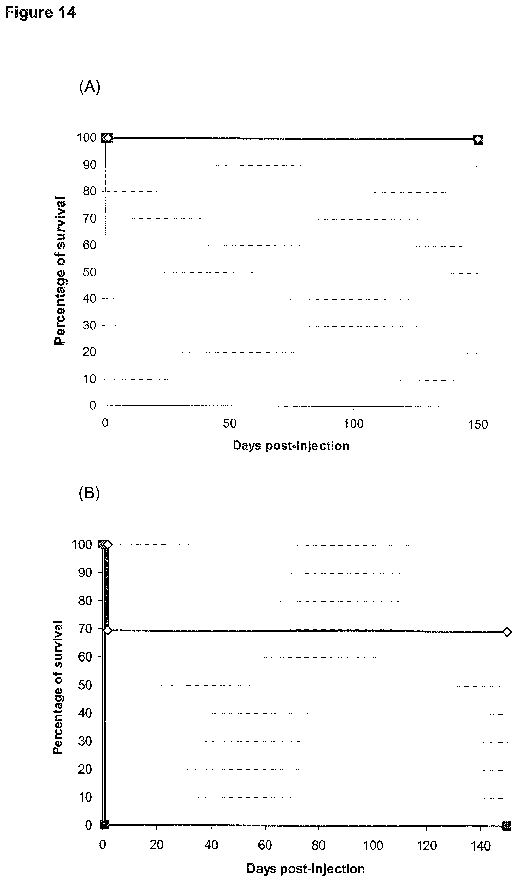

Viral pathogenicity was assessed with survival studies done on both Swiss nude mice (figure 13) and immunocompetents B6D2 mice (figure 14). Mice were injected I.V. with 1.10⁷ or 1.10⁸ PFU of all VVTK-/FCU1 and VVTK-I4L-/FCU1 in 100 µL of Buffer per mouse. Mice were observed daily throughout the course of the experiment. In Swiss nude mice (figure 13), the injection of 1x10⁸ PFU of VVTK-/FCU1 results in the death of 40% of the animals 3 days after infection. The remaining mice died between day 50 and day 80 after infection. The administration of VVTK-I4L-/FCU1 was less pathogenic, the majority of the animals died between day 65 to 140 (p<0.01). No evidence of toxicity has been observed with both viruses at 10⁷ pfu (figure 14 (A)). All mice died after i.v injection of 10⁸ pfu of VVTK-/FCU1 (figure 14 (B)). The group with treatment of VVTK-I4L-/FCU1 had significantly prolonged survival to 70% compared with the VVTK-/FCU1 infected mice (figure 14 (B)). Therefore, this result demonstrates the decrease of toxicity with the double-deleted virus VVTK-I4L-/FCU1.

### Pocks tail lesion model.

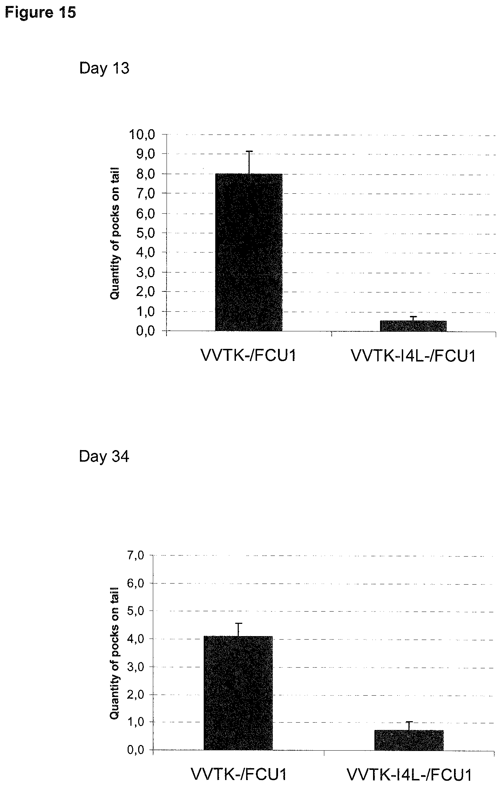

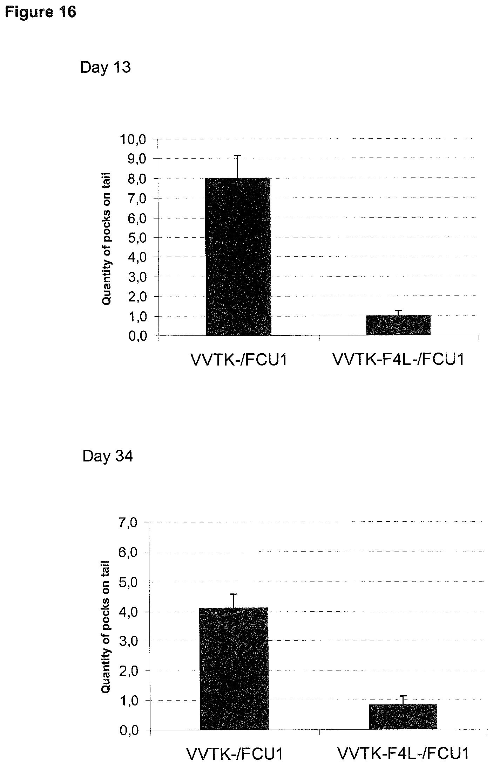

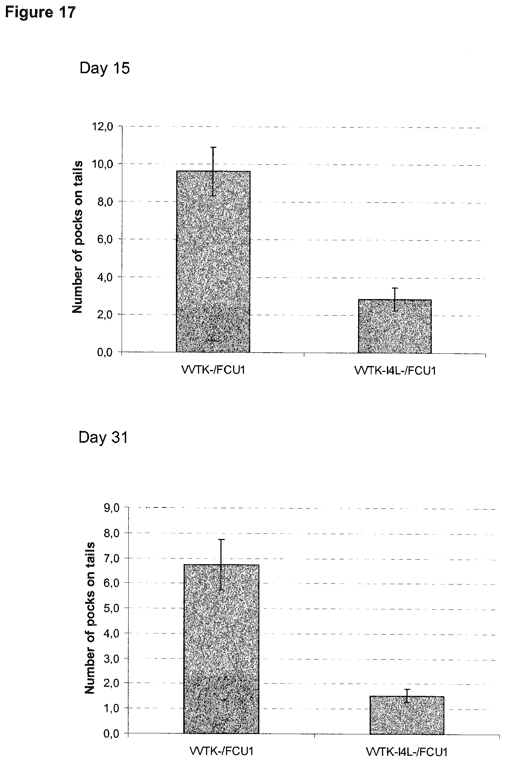

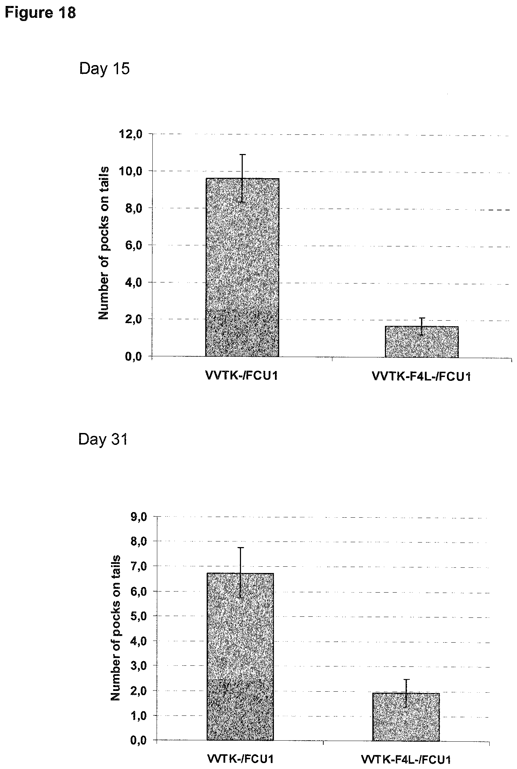

Swiss nude mice were injected I.V. with 1.10⁶ (figures 15 and 16) or 1.10⁷ (figures 17 and 18) PFU of each virus. Tail lesions were enumerated once a week. Mice injected with 1.10⁶ PFU of WTK-I4L-/FCU1 or VVTK-F4L-/FCU1 have less than 1 pock/mice compared with mice injected with VVTK-/FCU1 with a average of 8 pocks by mice in day 13 post-infection (p<0.001) as shown in figure 15 (A) and figure 16 (A). The results are similar at day 34 post-injection with an average of 4 pocks with VVTK-/FCU1 compared to nearly 1 for VVTK-I4L-/FCU1 or VVTK-F4L-/FCU1 (p<0.0001) as shown in figure 15 (B) and figure 16 (B). Mice injected with 1.10⁷ PFU of VVTK-I4L-/FCU1 or VVTK-F4L-/FCU1 have respectively an average of 3 pocks/mice and 2 pocks/mice compared to mice injected with 1.10⁷ PFU of VVTK-/FCU1 having an average of 10 pocks/mice at day 15 post-infection (figure 17 (A) and figure 18 (A)). At day 31 post-infection mice injected with VVTK-I4L-/FCU1 or VVTK-F4L-/FCU1 have respectively an average of 1,5 pock/mice and 2 pocks/mice compared to mice injected with VVTK-/FCU1 having an average of 7 pocks/mice (figure 17 (B) and figure 18 (B)). The difference in pock number between VVTK-/FCU1 and both VVTK-I4L-/FCU1 and VVTK-F4L-/FCU1 is statistically significant (p<0.01). The pocks formation is correlated with the replication of virus in the tail and so with virulence and toxicity. Injection in i.v of VVTK-I4L-/FCU1 or VVTK-F4L-/FCU1 is less toxic than with the single deleted TK virus.

### Combination VVTK-I4L-/FCU1 and irinotecan

The therapeutic effects of VVTK-I4L-/FCU1 treatment in combination with irinotecan were evaluated in vivo on human pancreas cancer MiaPaca2 cell line (ATCC® number: CRL-1420™) in the presence and absence of 5FC prodrug. More specifically, nude mice (n=12 mice/group) were injected subcutaneously with 5 x 106 MiaPaca2 cells. When the tumors had 100-200 mm3, VVTK-I4L-/FCU1 was injected intravenously at 1 x 106 PFU on days 24, 28 and 30 post tumor implantation. Irinotecan was administered intravenously at 33 mg/kg/day on days 28, 31, 35, 38, 42, 45. Starting on day 31 post tumor implantation, 5-FC was given by oral gavage at 100 mg/kg twice a day for 3 weeks. Tumor size was measured using calipers. Tumor volume was calculated in mm3 using the formula (π/6) (length x width2). Mice were killed when tumor size exceeded 4000 mm3 in volume. Mann-Whitney U-tests were used to determine tumor volume differences between the groups. A P-value <0.05 was considered significant.

Tumor volume of human pancreatic MiaPaca2 implanted tumors were measured regularly (every 4-6 days) after systemic injection of VVTK-I4L-/FCU1 +/- 5-FC and irinotecan over a period of 73 days post tumor implantation days. Table 6 provides the mean of tumor volumes in mm3 measured in the various mice groups at 24, 39, 50, 59, 63 and 73 days after implantation. "iri" represents irinotecan and "VV" VVTK-I4L-/FCU1.

**Table 6**

| Group | D24 | D39 | D50 | D59 | D63 | D73 |
|---|---|---|---|---|---|---|
| Control | 202 | 533 | 1395 | 1865 | 2876 | † |
| Iri | 195 | 245 | 122 | 118 | 157 | 719 |
| 5-FC | 218 | 561 | 1165 | 2484 | 2832 | † |
| Iri + 5FC | 192 | 217 | 113 | 197 | 253 | 634 |
| VV | 205 | 438 | 572 | 1371 | 1639 | 2265 |
| VV + 5FC | 189 | 217 | 88 | 74 | 71 | 66 |
| VV + iri | 192 | 303 | 490 | 1211 | 2067 | 2451 |
| VV + iri + 5FC | 197 | 225 | 78 | 69 | 71 | 96 |

| | | | | | | |
|---|---|---|---|---|---|---|
| †: Mice euthanized when tumor volume is >4000mm³. | | | | | | |

In MiaPaca2 model, in terms of tumor growth, there was no difference between the control (untreated) group and the group with 5-FC alone. Administration of VVTK-I4L-/FCU1 with or without 5-FC resulted in a slight antitumor effect. Administration of irinotecan with or without 5-FC displayed a significant tumor growth control compared with no treatment. The greatest benefit in terms of tumor growth control was seen with VVTK-I4L-/FCU1 in combination with irinotecan and this with or without 5-FC administration.
In conclusion, treatment with the combination of VVTK-I4L-/FCU1 and irinotecan (with or without 5-FC) resulted in significant inhibition of tumor growth compared with the other groups.

### Combination VVTK-I4L-/FCU1 and oxaliplatin

The therapeutic effects of VVTK-I4L-/FCU1 treatment in combination with oxaliplatin were evaluated in vivo on colorectal cancer LoVo cell line in the presence and absence of 5FC prodrug. 5FU was also tested in these conditions.

More specifically, nude mice (n=11 mice/group) were injected subcutaneously with 5 x 106 LoVo cells. When the tumors had 100-200 mm3, VVTK-I4L-/FCU1 was injected intravenously at 1 x 107 Pfu on day 21 post tumor implantation. Oxaliplatin was administered intraperitoneally at 2.5 mg/kg/day on days 24, 27, 31, 34, 38, 41. Starting on day 28 post tumor implantation, 5-FC was given by oral gavage at 100 mg/kg twice a day for 3 weeks. Starting on day 23 post tumor implantation, 5-FU was given intraperitoneally at 20 mg/kg/day for 3 weeks. Tumor size was measured using calipers. Tumor volume was calculated in mm3 using the formula (π/6) (length x width2). Mice were killed when tumor size exceeded 4000 mm3 in volume. Mann-Whitney U-tests were used to determine tumor volume differences between the groups. A P-value <0.05 was considered significant.

Tumor volume of human colorectal LoVo implanted tumors were measured regularly (every 4-6 days) after systemic injection of WTK-I4L-/FCU1 +/- 5-FC and oxaliplatin over a period of 52 days post tumor implantation days. Table 7 provides the mean of tumor volumes in mm3 measured in the various mice groups at 19, 25, 32, 46, 49 and 52 days after implantation. "oxa" represents oxaliplatin and "VV" VVTK-I4L-/FCU1.

**Table 7**

| Group | D19 | D25 | D32 | D46 | D49 | D52 |
|---|---|---|---|---|---|---|
| Control | 167 | 379 | 746 | 1849 | 2077 | 2558 |
| oxa | 172 | 283 | 529 | 1313 | 1499 | 1768 |
| 5-FC | 169 | 341 | 592 | 1511 | 1786 | 1987 |
| oxa + 5FC | 176 | 349 | 682 | 1476 | 1899 | 1828 |
| 5-FU | 169 | 293 | 508 | 1362 | 1759 | 1558 |
| Oxa + 5FU | 179 | 309 | 517 | 1141 | 1323 | 1558 |
| VV | 175 | 315 | 579 | 1554 | 1830 | 1724 |
| VV + 5FC | 172 | 316 | 573 | 790 | 836 | 974 |
| VV + oxa | 172 | 355 | 555 | 1024 | 1089 | 1215 |
| VV + oxa + 5FC | 165 | 328 | 559 | 657 | 623 | 760 |

In LoVo model, in terms of tumor growth, treatment with oxaliplatin alone, 5-FC alone, oxaliplatin + 5-FC, 5-FU alone, oxaliplatin + 5-FU or VVTK-I4L-/FCU1 alone provided a slight control of tumor growth compared with control (untreated) mice. VVTK-I4L-/FCU1 + oxaliplatin or VVTK-I4L-/FCU1 + 5-FC resulted in significant tumor growth control compared with no treatment. The greatest benefit in terms of tumor growth control was seen with the combination VVTK-I4L-/FCU1 + 5-FC + oxaliplatin. Treatment with this combination resulted in significant inhibition of tumor growth compared with no treatment, oxaliplatin alone, 5-FC alone, oxaliplatin + 5-FC, VVTK-I4L-/FCU1 alone, VVTK-I4L-/FCU1 + oxaliplatin or VVTK-I4L-/FCU1 + 5-FC. Moreover the treatment with the combination VVTK-I4L-/FCU1 + 5-FC + oxaliplatin resulted in significantly antitumor effect compared with the chemotherapeutic drug 5-FU alone or in combination with oxaliplatin (5-FU was given intraperitoneally at the maximum tolerated concentrations).

In conclusion, treatment with the combination of VVTK-I4L-/FCU1 and oxaliplatin notably in presence of 5-FC proved to provide an enhanced antitumor effect as compared to each of VVTK-I4L-/FCU1 and oxaliplatin as well as to the standard antitumor drug 5FU.

### Statistical analysis.

Statistical analyses were performed using the nonparametric Mann-Whitney *U*test and STATISTICA 7.1 software (StatSoft, Inc.). A *P<* 0.05 was considered to be statistically significant.

### References

- US 5364773 (VIROGENETICS CORPORATION (TROY, NY)) 15.11.1994
- WO 2004/014314 (KIRN DAVID (US)) 19.02.2004
- WO 2004/014314 (KIRN DAVID (US)) 19.02.2004
- US 5364773 (VIROGENETICS CORPORATION (TROY, NY)) 15.11.1994
- WO 93/01281 (US HEALTH)
- WO 2005/007857
- WO 2005/007857
- EP 0998568 A
- EP 0998568 A
- EP 0998568 A
- EP 0998568 A
- WO 96/16183
- EP 0998568 A
- EP 0998568 A
- WO 2006/048768
- HERMISTON. A demand for next-generation oncolytic adenoviruses. Current opinion in molecular therapeutics. 2006, vol.8, no.4, p.322-30.
- FISHER. Striking out at disseminated metastases: the systemic delivery of oncolytic viruses. Current opinion in molecular therapeutics 2006, vol.8, no.4, p.301-13.
- CHERNAJOVSKY, et al. Fighting cancer with oncolytic viruses. British medical journal. 2006, vol.332, no.7534, p.170-2.
- JIANG, et al. Oncolytic adenoviruses as antiglioma agents. Expert review of anticancer therapy. 2006, vol.6, no.5, p.697-708.
- COHEN, et al. ONYX-015. Onyx Pharmaceuticals. Current opinion in investigational drugs. 2001, vol.2, no.12, p.1770-5.
- ROBERTS, et al. Naturally oncolytic viruses. Current opinion in molecular therapeutics. 2006, vol.8, no.4, p.314-21.
- THORNE, et al. Vaccinia virus and oncolytic virotherapy of cancer. Current opinion in molecular therapeutics. 2005, vol.7, no.4, p.359-65.
- STANFORD, et al. Myxoma virus and oncolytic virotherapy: a new biologic weapon in the war against cancer. Expert opinion on biological therapy. 2007, vol.7, no.9, p.1415-25.
- CONO, et al. Smallpox vaccination and adverse reactions. Guidance for clinicians. MMWR. Recommendations and reports: Morbidity and mortality weekly report. Recommendations and reports / Centers for Disease Control. 2003, vol.52, no.RR-4, p.1-28.
- XIANGZHI, et al. Vaccinia virus K1L protein supports viral replication in human and rabbit cells through a cell-type-specific set of its ankyrin repeat residues that are distinct from its binding site for ACAP2. Journal of virology. 2006, vol.353, no.1, p.220-233.
- MCCART, et al. Systemic cancer therapy with a tumor selective vaccinia virus mutant lacking thymidine kinase and vaccinia growth factor genes.. Cancer res.. 2001, no.61, p.8751-57.
- KIM, et al. Systemic armed oncolytic ans immunologic therapy for cancer with JX-594, a targeted poxvirus expressing GM-CSF. Molecular Therapeutic. 2006, no.14, p.361-70.
- SLABAUGH, et al.. Journal of virology. 1988, vol.62, p.519-27.
- TENGELSEN,et al.. Virology. 1988, no.164, p.121-31.
- SCHMITT, et al.. Journal of virology. 1988, no.62, p.1889-97.
- SLABAUGH, et al.. Journal of virology. 1984, no.52, p.507-14.
- SLABAUGH, et al.. Journal of virology. 1984, no.52, p.501-6.
- HOWELL, et al.. Journal of Biological Chemistry. 1992, no.267, p.1705-11.
- ANTOINE. . Virology. 1998, no.244, p.365-396.
- EL OMARI, et al. Structure of vaccinia virus thymidine kinase in complex with dTTP: insights for drug design. BMC structural biology. 2006, no.6, p.22.
- MCCART, et al. Systemic cancer therapy with a tumor-selective vaccinia virus mutant lacking thymidine kinase and vaccinia growth factor genes. cancer research. 2001, vol.61, no.24, p.8751-7.
- PUHLMANN, et al. Vaccinia as a vector for tumor-directed gene therapy: biodistribution of a thymidine kinase-deleted mutant. Cancer gene therapy. 2000, vol.7, no.1, p.66-73.
- GNANT, et al. Systemic administration of a recombinant vaccinia virus expressing the cytosine deaminase gene and subsequent treatment with 5-fluorocytosine leads to tumor-specific gene expression and prolongation of survival in mice. Cancer Research. 1999, vol.59, no.14, p.3396-403.
- MCGEOGH. . Nucleic AcidsResearch. 1990, no.18, p.4105-10.
- BROYLES. . Virology. 1993, no.195, p.863-5.
- JUND, et al.. Journal of Bacteriology. 1970, no.102, p.607-15.
- BECK, etal.. Journal of Bacteriology. 1972, no.110, p.219-28.
- HOEPRICH, et al.. Journal of Infectious Diseases. 1974, no.130, p.112-18.
- ESDERS, et al.. J. biol. chem.. 1985, no.260, p.3915-22.
- KOECHLIN, et al.. Biochemical pharmacology. 1966, no.15, p.435-46.
- POLAK, et al.. Chemotherapy. 1976, no.22, p.137-53.
- JUND, et al.. Journal of Bacteriology.1970, no.102, p.607-15.
- KILLSTRUP, et al.. Journal of Bacteriology. 1989, no.171, p.2124-2127.
- HUBER, et al.. Cancer Research. 1993, no.53, p.4619-4626.
- MULLEN, et al.. Proceedings of the National Academyof Sciences of the United States of America. 1992, no.89, p.33-37.
- HUBER, et al.. Proceedings of the NationalAcademy of Sciences of the United States of America. 1994, no.91, p.8302-6.
- MESNIL, et al.. Proceedings of the National Academy of Sciences of the United States of America. 1996, no.93, p.1831-35.
- ANDERSEN, et al. Characterization of the upp gene encoding uracil phosphoribosyltransferase of Escherichia coli K12. European Journal of Biochemistry. 1992, no.204, p.51-56.
- MARTINUSSEN, et al. Cloning and characterization of upp, a gene encoding uracil phosphoribosyltransferase from Lactococcus lactis. Journal of Bacteriology. 1994, vol.176, no.21, p.6457-63.
- KIM, et al. Complete sequence of the UPP gene encoding uracil phosphoribosyltransferase from Mycobacterium bovis BCG. Biochemistry and molecular biology international. 1997, vol.41, no.6, p.1117-24.
- MARTINUSSEN, et al. Two genes encoding uracil phosphoribosyltransferase are present in Bacillus subtilis. Journal of Bacteriology. 1995, vol.177, no.1, p.271-4.
- KERN, et al. The FUR1 gene of Saccharomyces cerevisiae: cloning, structure and expression of wild-type and mutant alleles. Gene. 1990, vol.88, no.2, p.149-57.
- CHAKRABARTI. . Biotechniques. 1997, no.23, p.1094-97.
- HAMMOND, et al. . Journal of VirologicalMethods. 1997, no.66, p.135-38.
- KUMAR.. Virology. 1990, no.179, p.151-8.

## Claims

1. A composition comprising an oncolytic poxvirus comprising a defective gene encoding the large subunit of ribonucleotide reductase (I4L) and/or the small subunit of ribonucleotide reductase (F4L) and a defective gene encoding thymidine kinase (J2R) and further comprising a suicide gene for use in treating cancer in combination with one or more substances effective in anticancer therapy, wherein the one or more substances effective in anticancer therapy are selected from irinotecan and oxaliplatin.

2. The composition for use in treating cancer according to claim 1 wherein said poxvirus is a Vaccinia virus strain Copenhagen.

3. The composition for use in treating cancer according to any one of claims 1 or 2 wherein said composition and said one or more substances are administered via the systemic route or into the tumor, preferably intravenously.

4. The composition for use in treating cancer according to anyone of claims 1 to 3 wherein doses of irinotecan are comprised from 5 to 100 mg/kg/day and doses of poxvirus are comprised from 1x10⁶ to 1x10⁹ pfu.

5. The composition for use in treating cancer according to claim 4 wherein said poxvirus is injected one to three times intravenously and irinotecan is injected 3 to 10 days after the first administration of said poxvirus.

6. The composition for use in treating cancer according to any one of claims 1 to 3 wherein oxaliplatin is administered intravenously or intraperitoneally and poxvirus is administered intravenously.

7. The composition for use in treating cancer according to claim 6 wherein doses of oxaliplatin are comprised from 0.5 to 10 mg/kg/day and doses of poxvirus are comprised from 1x10⁶ to 1x10⁹ pfu.

8. The composition for use in treating cancer according to claim 6 or 7 wherein said poxvirus is injected once to three times intravenously and oxaliplatin every two weeks for one or more cycles.

9. The composition for use in treating cancer according to claim 8 wherein said oxaliplatin is administered 3 to 10 days after the first administration of said poxvirus composition.

10. The composition for use in treating cancer according to anyone of claims 1 to 9 wherein said suicide gene codes a protein having at least one cytosine deaminase and one uracil phosphoribosyl transferase activity.

11. The composition for use in treating cancer according to anyone of claims 1 to 9 wherein the suicide gene encodes a polypeptide comprising an amino acid sequence substantially as represented in the sequence in the sequence identifier SEQ ID NO: 1 or SEQ ID NO: 3.

12. The composition for use in treating cancer according to claim 10 or 11 wherein said use further comprises a pharmaceutically acceptable quantity of a prodrug.

13. The composition for use in treating cancer according to claim 12 wherein said prodrug is 5-fluorocytosine.

14. The composition for use in treating cancer according to claim 13 wherein said prodrug is administered preferably at least 3 days, more preferably at least 4 days and even more preferably at least 5 days after the administration of said poxvirus composition.

15. The composition for use in treating cancer according to anyone of claims 1 to 14 wherein said cancer is selected from the group consisting of cancers of the breast, of the uterus, of the prostate, of the lung, of the bladder, of the liver, of the colon, of the pancreas, of the stomach, of the esophagus, of the larynx, of the central nervous system, e.g. glioblastoma, and of the blood, preferably pancreas cancer and colorectal cancer.

## Patentansprüche

1. Zusammensetzung, die ein onkolytisches Pockenvirus umfasst, das ein defektes Gen umfasst, das die große Untereinheit der Ribonucleotid-Reduktase (I4L) und/oder die kleine Untereinheit der Ribonucleotid-Reduktase (F4L) codiert, und ein defektes Gen umfasst, das Thymidin-Kinase (J2R) codiert, und des Weiteren ein Suizid-Gen (suicide gene) umfasst, zur Verwendung bei der Behandlung von Krebs in Kombination mit einer oder mehreren Substanz(en), die bei der Anti-Krebs-Therapie wirksam sind, wobei die eine oder mehreren Substanz(en), die bei der Anti-Krebs-Therapie wirksam sind, ausgewählt ist/sind aus Irinotecan und Oxaliplatin.

2. Zusammensetzung zur Verwendung bei der Behandlung von Krebs nach Anspruch 1, wobei das Pockenvirus ein Vaccinia-Virus vom Stamm Copenhagen ist.

3. Zusammensetzung zur Verwendung bei der Behandlung von Krebs nach Anspruch 1 oder 2, wobei die Zusammensetzung und die eine oder mehreren Substanz(en) über den systemischen Weg oder in den Tumor, vorzugsweise intravenös, verabreicht wird/werden.

4. Zusammensetzung zur Verwendung bei der Behandlung von Krebs nach einem der Ansprüche 1 bis 3, wobei die Dosen von Irinotecan von 5 bis 100 mg/kg/Tag umfassen und Dosen des Pockenvirus von 1x10⁶ bis 1x10⁹ pfu umfassen.

5. Zusammensetzung zur Verwendung bei der Behandlung von Krebs nach Anspruch 4, wobei das Pockenvirus einmal bis dreimal intravenös injiziert und Irinotecan 3 bis 10 Tage nach der ersten Verabreichung des Pockenvirus injiziert wird.

6. Zusammensetzung zur Verwendung bei der Behandlung von Krebs nach einem der Ansprüche 1 bis 3, wobei Oxaliplatin intravenös oder intraperitoneal verabreicht und das Pockenvirus intravenös verabreicht wird.

7. Zusammensetzung zur Verwendung bei der Behandlung von Krebs nach Anspruch 6, wobei Dosen von Oxaliplatin von 0,5 bis 10 mg/kg/Tag umfassen und Dosen von Pockenvirus von 1x10⁶ bis 1x10⁹ pfu umfassen.

8. Zusammensetzung zur Verwendung bei der Behandlung von Krebs nach Anspruch 6 oder 7, wobei das Pockenvirus einmal bis dreimal intravenös und Oxaliplatin alle zwei Wochen für einen oder mehrere Zyklen injiziert wird.

9. Zusammensetzung zur Verwendung bei der Behandlung von Krebs nach Anspruch 8, wobei das Oxaliplatin 3 bis 10 Tage nach der ersten Verabreichung der Pockenvirus-Zusammensetzung verabreicht wird.

10. Zusammensetzung zur Verwendung bei der Behandlung von Krebs nach einem der Ansprüche 1 bis 9, wobei das Suizid-Gen ein Protein codiert, das mindestens eine Cytosin-Desaminase- und eine Uracil-Phosphoribosyl-Transferase-Aktivität hat.

11. Zusammensetzung zur Verwendung bei der Behandlung von Krebs nach einem der Ansprüche 1 bis 9, wobei das Suizid-Gen ein Polypeptid codiert, das eine wie im Wesentlichen in der Sequenz in der Sequenzkennzahl SEQ ID NO:1 oder SEQ ID NO:3 dargestellten Aminosäuresequenz umfasst.

12. Zusammensetzung zur Verwendung bei der Behandlung von Krebs nach Anspruch 10 oder 11, wobei die Verwendung des Weiteren eine pharmazeutisch verträgliche Menge eines Arzneistoffvorläufers (Prodrug) umfasst.

13. Zusammensetzung zur Verwendung bei der Behandlung von Krebs nach Anspruch 12, wobei der Arzneistoffvorläufer (Prodrug) 5-Fluorcytosin ist.

14. Zusammensetzung zur Verwendung bei der Behandlung von Krebs nach Anspruch 13, wobei der Arzneistoffvorläufer (Prodrug) vorzugsweise mindestens drei Tage, mehr bevorzugt mindestens 4 Tage und noch bevorzugter mindestens 5 Tage nach der Verabreichung der Pockenvirus-Zusammensetzung verabreicht wird.

15. Zusammensetzung zur Verwendung bei der Behandlung von Krebs nach einem der Ansprüche 1 bis 14, wobei der Krebs ausgewählt ist aus der Gruppe bestehend aus Brust-, Gebärmutter-, Prostata-, Lungen-, Blasen-, Leber-, Dickdarm-, Pankreas-, Magen-, Speiseröhren-, Kehlkopfkrebs, Krebs des zentralen Nervensystems, z.B. Glioblastoma, und Blutkrebs, vorzugsweise Bauchspeicheldrüsenkrebs und Dickdarmkrebs.

## Revendications

1. Composition comprenant un poxvirus oncolytique comprenant un gène défectueux codant la large sous-unité de ribonucléotide réductase (I4L) et/ou la petite sous-unité de ribonucléotide réductase (F4L) et un gène défectueux codant la thymidine kinase (J2R), et comprenant en outre un gène suicide, pour une utilisation dans le traitement d'un cancer en combinaison avec une ou plusieurs substances efficaces en thérapie anticancéreuse, dans laquelle la ou les substances efficaces en thérapie anticancéreuse sont choisies parmi l'irinotécan et l'oxaliplatine.

2. Composition pour une utilisation dans le traitement d'un cancer selon la revendication 1, dans laquelle ledit poxvirus est la souche Copenhague du virus de la vaccine.

3. Composition pour une utilisation dans le traitement d'un cancer selon l'une quelconque des revendications 1 et 2, dans laquelle ladite composition et lesdites une ou plusieurs substances sont administrées par voie systémique ou dans la tumeur, de préférence par voie intraveineuse.

4. Composition pour une utilisation dans le traitement d'un cancer selon l'une quelconque des revendications 1 à 3, dans laquelle les doses d'irinotécan sont comprises entre 5 et 100 mg/kg/jour et les doses de poxvirus sont comprises entre 1 x 10⁶ et 1 x 10⁹ pfu.

5. Composition pour une utilisation dans le traitement d'un cancer selon la revendication 4, dans laquelle ledit poxvirus est injecté une à trois fois par voie intraveineuse et l'irinotécan est injecté 3 à 10 jours après la première administration dudit poxvirus.

6. Composition pour une utilisation dans le traitement d'un cancer selon l'une quelconque des revendications 1 à 3, dans laquelle l'oxaliplatine est administré par voie intraveineuse ou intrapéritonéale et le poxvirus est administré par voie intraveineuse.

7. Composition pour une utilisation dans le traitement d'un cancer selon la revendication 6, dans laquelle les doses d'oxaliplatine sont comprises entre 0,5 et 10 mg/kg/jour et les doses de poxvirus sont comprises entre 1 x 10⁶ et 1 x 10⁹ pfu.

8. Composition pour une utilisation dans le traitement d'un cancer selon la revendication 6 ou 7, dans laquelle ledit poxvirus est injecté une à trois fois par voie intraveineuse et l'oxaliplatine toutes les deux semaines sur un ou plusieurs cycles.

9. Composition pour une utilisation dans le traitement d'un cancer selon la revendication 8, dans laquelle ledit oxaliplatine est administré 3 à 10 jours après la première administration de ladite composition de poxvirus.

10. Composition pour une utilisation dans le traitement d'un cancer selon l'une quelconque des revendications 1 à 9, dans laquelle ledit gène suicide code une protéine ayant au moins une activité de cytosine désaminase et au moins une activité d'uracile phosphoribosyle transférase.

11. Composition pour une utilisation dans le traitement d'un cancer selon l'une quelconque des revendications 1 à 9, dans laquelle ledit gène suicide code un polypeptide comprenant une séquence d'acides aminés sensiblement telle que représentée dans la séquence de l'identifiant de séquence SEQ ID NO : 1 ou SEQ ID NO : 3.

12. Composition pour une utilisation dans le traitement d'un cancer selon la revendication 10 ou 11, dans laquelle ladite utilisation comprend en outre une quantité pharmaceutiquement acceptable d'un promédicament.

13. Composition pour une utilisation dans le traitement d'un cancer selon la revendication 12, dans laquelle ledit promédicament est une 5-fluorocytosine.

14. Composition pour une utilisation dans le traitement d'un cancer selon la revendication 13, dans laquelle ledit promédicament est administré de préférence au moins 3 jours, préférentiellement au moins 4 jours et de manière préférée au moins 5 jours après l'administration de ladite composition de poxvirus.

15. Composition pour une utilisation dans le traitement d'un cancer selon l'une quelconque des revendications 1 à 14, dans laquelle ledit cancer est choisi dans le groupe constitué par les cancers du sein, de l'utérus, de la prostate, du poumon, de la vessie, du foie, du côlon, du pancréas, de l'estomac, de l'œsophage, du larynx, du système nerveux central, par exemple un glioblastome, et du sang, de préférence un cancer du pancréas et un cancer colorectal.
